# EUROPEAN PATENT SPECIFICATION

(11) **EP 1 909 811 B1**
(45) Date of publication and mention of the grant of the patent: **01.02.2012**
(21) Application number: 06755670.4
(22) Date of filing: 28.06.2006
(51) Int. Cl.: A61K 38/03, A61P 35/00

(54) **HOX PEPTIDES AS PBX MODULATORS FOR THE TREATMENT OF CANCER**
HOX PEPTIDE ALS PBX-MODULATOREN ZUR BEHANDLUNG VON KREBS
PEPTIDES FRAGMENTS DE HOX COMME MODULATEURS DE PBX POUR LE TRAITEMENT DU CANCER

(30) Priority: 29.06.2005 US 695136 P
(43) Date of publication of application: 16.04.2008
(73) Proprietor: St. George's Enterprises Limited, London SW17 0RE (GB)
(72) Inventor: MORGAN, Richard George L. Dpt of Basic Med Science, Tooting London SW17 0RE (GB); SOHAL, Jastinder Department of Basic Med. Science, Tooting London SW17 0RE (GB)
(74) Representative: Woods, Geoffrey Corlett
(86) International application number: PCT/GB2006/002390
(87) International publication number: WO 2007/000601

(56) References cited:
- WO-A-97/42222
- WO-A-2004/055049

### SEQUENCE LISTING

<110> St. George's Enterprises Limited
<120> Gene Regulation
<130> N94942A GCW
<160> 11
<170> PatentIn version 3.2
<210> 1
   <211> 10
   <212> PRT
   <213> Artificial Sequence
<220>
   <223> Synthetic peptide
<220>
   <221> MISC_FEATURE
   <222> (1)..(1)
   <223> Xaa may be present or absent, is one or more amino acids, and is preferably W, T, PE, KQI, W, PQT, H or RI
<220>
   <221> MISC_FEATURE
   <222> (2)..(2)
   <223> Xaa is an amino acid with an aromatic side chain, preferably Y, F or W
<220>
   <221> MISC_FEATURE
   <222> (3)..(3)
   <223> Xaa is the amino acid P or D
<220>
   <221> MISC_FEATURE
   <222> (6)..(6)
   <223> Xaa is an amino acid with a basic side chain, preferably K, R or H
<220> .
   <221> MISC_FEATURE
   <222> (7)..(7)
   <223> Xaa is an amino acid with a charged side chain, preferably a basic side chain, especially preferably K, R, E, H, D, N or Q
<220>
   <221> MISC_FEATURE
   <222> (8)..()
   <223> Xaa is an amino acid with a charged side chain, preferably a basic side chain, especially preferably K, R, E, H, D, N or Q
<220>
   <221> MISC_FEATURE
   <222> (9)..(9)
   <223> Xaa is an amino acid with a basic side chain or serine, especially preferably H, S, R or K
<220>
   <221> MISC_FEATURE
   <222> (10)..(10)
   <223> Xaa is or comprises a cationic polymer of basic amino acids
<400> 1
   Xaa Xaa Xaa Trp Met Xaa Xaa Xaa Xaa Xaa
   1               5                   10
<210> 2
   <211> 10
   <212> PRT
   <213> Artificial Sequence
<220>
   <223> Synthetic peptide
<220>
   <221> MISC_FEATURE
   <222> (1)..(1)
   <223> Xaa may be present or absent, is one or more amino acids, and is preferably W, T, PE, KQI, W, PQT, H or RI
<220>
   <221> MISC_FEATURE
   <222> (2)..(2)
   <223> Xaa is Y or F
<220>
   <221> MISC_FEATURE
   <222> (3)..(3)
   <223> Xaa is P or D
<220>
   <221> MISC_FEATURE
   <222> (6)..(6)
   <223> Xaa is K or R
<220>
   <221> MISC_FEATURE
   <222> (7)..(7)
   <223> Xaa is K, R or E
<220>
   <221> MISC_FEATURE
   <222> (8)..(8)
   <223> Xaa is H, R, Q or K
<220>
   <221> MISC_FEATURE
   <222> (9)..(9)
   <223> Xaa is H, S, R or K
<220>
   <221> MISC_FEATURE
   <222> (10)..(10)
   <223> Xaa is or comprises a cationic polymer of basic amino acids
<400> 2
   Xaa Xaa Xaa Trp Met Xaa Xaa Xaa Xaa Xaa
   1               5                   10
<210> 3
   <211> 10
   <212> PRT
   <213> Artificial Sequence
<220>
   <223> Synthetic peptide
<220>
   <221> MISC_FEATURE
   <222> (1) .. (1)
   <223> Xaa may be present or absent, is one or more amino acids, and is preferably W, T, PE, KQI, VV, PQT, H or RI
<220>
   <221> MISC_FEATURE
   <222> (6) .. (6)
   <223> Xaa is K or R
<220>
   <221> MISC_FEATURE
   <222> (7) ..(7)
   <223> Xaa is K, R or E
<220>
   <221> MISC_FEATURE
   <222> (8) .. (8)
   <223> Xaa is H, R, Q or K
<220>
   <221> MISC_FEATURE
   <222> (9) .. (9)
   <223> Xaa is H, S, R or K
<220>
   <221> MISC_FEATURE
   <222> (10) .. (10)
   <223> Xaa is is or comprises a cationic polymer of basic amino acids
<400> 3
   Xaa Tyr Pro Trp Met Xaa Xaa Xaa Xaa Xaa
   1               5                   10
<210> 4
   <211> 10
   <212> PRT
   <213> Artificial Sequence
<220>
   <223> Synthetic peptide
<220>
   <221> MISC_FEATURE
   <222> (10) ..(10)
   <223> Xaa is or comprises a cationic polymer of basic amino acids
<400> 4
   Trp Tyr Pro Trp Met Lys Lys His His Xaa
   1               5                   10
<210> 5
   <211> 9
   <212> PRT
   <213> Artificial Sequence
<220>
   <223> Synthetic peptide
<400> 5
   Arg Arg Arg Arg Arg Arg Arg Arg Arg
   1               5
<210> 6
   <211> 18
   <212> PRT
   <213> Artificial Sequence
<220>
   <223> Synthetic peptide
<400> 6
   Trp Tyr Pro Trp Met Lys Lys His His Arg Arg Arg Arg Arg Arg Arg
   1               5                   10                  15
   Arg Arg
<210> 7
   <211> 9
   <212> PRT
   <213> Artificial Sequence
<220>
   <223> Synthetic peptide
<400> 7
   Trp Tyr Pro Trp Met Lys Lys His His
   1               5
<210> 8
   <211> 24
   <212> PRT
   <213> Artificial Sequence
<220>
   <223> Synthetic peptide
<400> 8
   Trp Tyr Pro Trp Met Lys Lys His His Arg Gln Ile Lys Ile Trp Phe
   1               5                   10                  15
   Gln Asn Arg Arg Met Lys Trp Lys
               20
<210> 9
   <211> 18
   <212> PRT
   <213> Artificial Sequence
<220>
   <223> Synthetic peptide
<400> 9
   Trp Cys Pro Trp Leu Asp Arg His Gly Arg Arg Arg Arg Arg Arg Arg
   1               5                   10                  15
   Arg Arg
<210> 10
   <211> 18
   <212> PRT
   <213> Artificial Sequence
<220>
   <223> Synthetic peptide
<400> 10
   Met Cys Ala Ala Gly Asp Asp Ala Leu Arg Arg Arg Arg Arg Arg Arg
   1               5                   10                  15
   Arg Arg
<210> 11
   <211> 18
   <212> PRT
   <213> Artificial Sequence
<220>
   <223> Synthetic peptide
<220>
   <221> MISC_FEATURE
   <222> (1) .. (1)
   <223> W is a D isomer
<220>
   <221> MISC_FEATURE
   <222> (18) .. (18)
   <223> R is a D isomer
<400> 11
   Trp Tyr Pro Trp Met Lys Lys His His Arg Arg Arg Arg Arg Arg Arg
   1               5                   10                  15
   Arg Arg

## Description

### Field of the Invention

The present invention relates to molecules which impair PBX-dependent transcription regulation, particularly peptides which affect the binding of HOX to PBX and their use in a number of applications, including the reduction of aberrant cell division, e.g. to treat certain cancers, and to maintain pluripotency of stem cells, e.g. to maintain the pluripotency of stem cells during culture expansion.

### Background of the Invention

A variety of transcription factors are involved in the regulation of expression of proteins during embryogenesis and adult stem cell maturation. Homeobox (HOX) genes contain a highly conserved nucleotide sequence of about 180bp which encodes a homeodomain of about 60 amino acids. A homeodomain is a DNA-binding protein domain which can bind to target sequences in other genes and regulate their expression during development. The clustered Hox genes are key developmental regulators and are highly conserved throughout evolution. The homeotic HOX proteins which they encode function as transcription factors to control axial patterning by regulating the transcription of subordinate downstream genes, e.g. developmental genes

Pre-B-cell transformation related gene (PBX) is also an important regulatory protein that controls gene expression during development by interacting cooperatively with HOX to bind to the target DNA (Mann et al., 1996, Trends Genet., 12(7), p258-262).

### Summary of the Invention

The present inventors have developed peptides which mimic the region of HOX to which PBX binds and act as antagonists of that binding. These peptides are based on the hexapeptide region of the HOX protein HOXB-4.

It has also been found that by inhibiting the binding of PBX to its binding partners, aberrant cell growth may be reduced to prevent or treat disorders or conditions in which such cell growth occurs. Such inhibition has been found to have profound and useful effects on stem cells, which allows the pluripotency of these cells to be maintained. These findings offer significant clinical applications in which desired cells may be protected and possibly expanded whilst the growth of detrimental cells may be prevented.

Accordingly the invention relates to peptides comprising the amino acid sequence (I):

Y₁ X₁ X₂ X₃ W X₄ X₅ X₆ X₇ X₈ Y₂ (I)

wherein
X₁ is W;
X₂ is Y;
X₃ is P;
X₄ is M, I, V or L;
X₅ is K or R;
X₆ is K or R;
X₇ is H or T;
X₈ is one or more amino acids or absent; and

Y₁ and Y₂ are each either absent or (Arg)₉ such that at least one of Y₁ and Y₂ is present.

Optionally at least the N-terminal and C-terminal amino acids of said peptide may be in the D-conformation.

Peptides of the invention may be used for the treatment or prevention of a disorder in which aberrant cell division occurs. The disorder to be treated is preferably a cancer. The cells to be treated preferably express one or more Hox genes.

The invention also provides:
- use of a peptide of the invention and a cytotoxic or chemotherapeutic agent in the manufacture of a medicament for simultaneous, sequential or separate use in the treatment or prevention of a disorder in which aberrant cell division occurs.
- a peptide of the invention for use in reducing the side effects of a cytotoxic or chemotherapeutic agent.
- the invention can be used for treating a disorder in which aberrant cell division occurs in a human or animal comprising administering to said human or animal a therapeutically effective amount of a peptide of the invention.
- a method of maintaining or expanding stem cells *ex vivo* comprising contacting said stem cells with a peptide of the invention. Such a method may further comprise the step of culturing said cells in the absence of said peptide, and/or said stem cells can be administered to a patient in need thereof. Stem cells that have been maintained or expanded by such a method are also described herein.

Such a stem cell can be used for the treatment or prevention of a condition resulting in a decreased level of stem cells.
- a pharmaceutical composition comprising a peptide of the invention and a pharmaceutically acceptable carrier.

### Description of the Figures

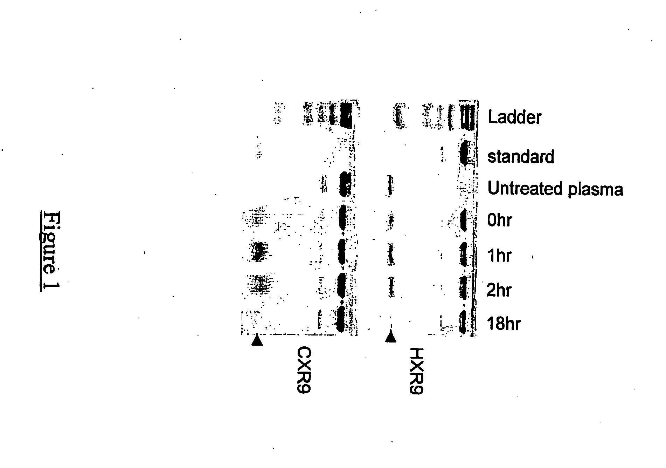

Figure 1 shows the stability of the HXArg9(2D) (HXR9) and CX2Arg9(2D) (CXR9) peptides in human plasma. Peptides were incubated in human serum for the times shown and then visualised on a Coomassie brilliant blue stained gel. The arrow heads indicate the position of intact peptide. Standard = peptide only in this lane as a marker.

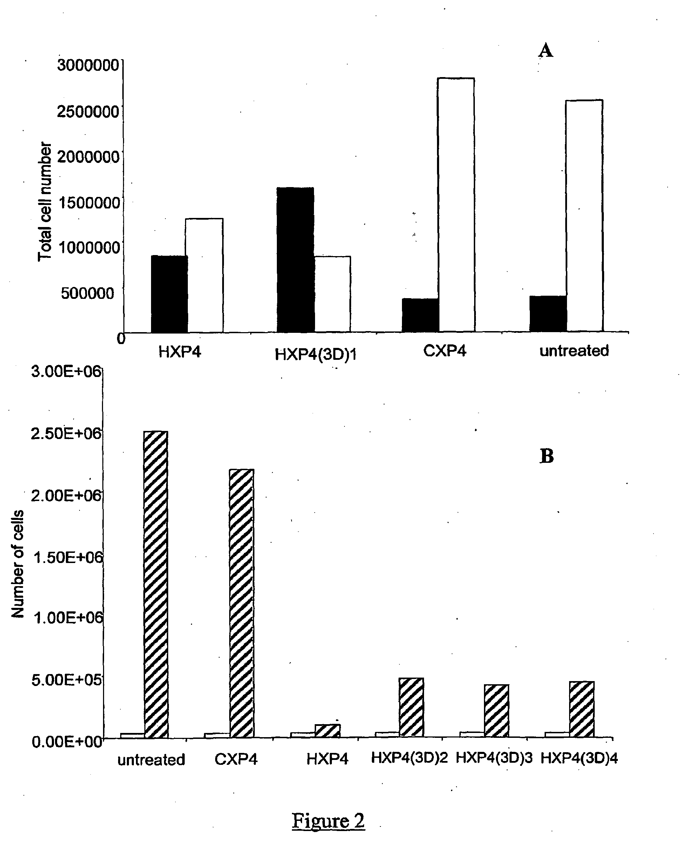

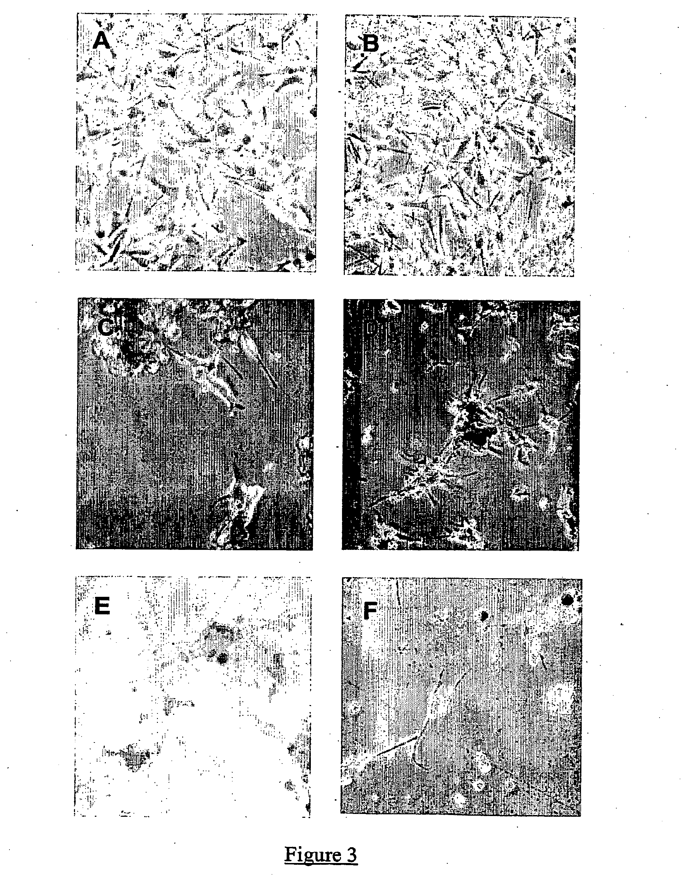

Figures 2 and 3 show the effect of peptides on B16 murine melanoma cells.

In Figure 2A the peptides as indicated were applied to the cells at a concentration of 60µM. The number of living and dead cells were assessed after 3 hours. Black bars = number of cells dead. White bars = number of cells alive.

In Figure 2B the peptides as indicated were applied to the cells at a concentration of 200µg/ml for 4 hours. The number of cells present was assessed on the day of treatment (white bars) and on day 4 (diagonal stripes).

Figure 3 shows representative cells from the experiments of Figure 2B. A = untreated; B = CXP4; C = HXP4; D = HXP4(3D)2; E = HXP4(3D)3; F = HXP4(3D)4.

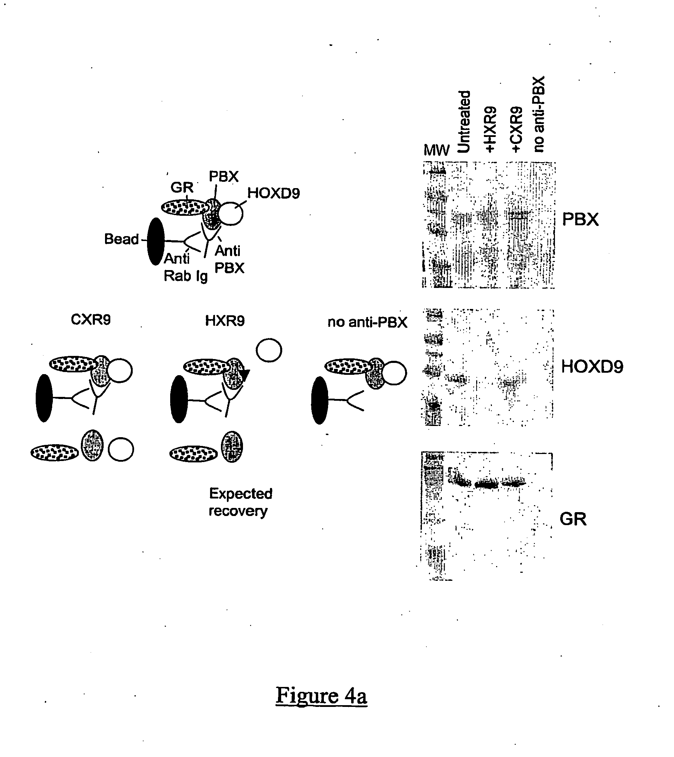

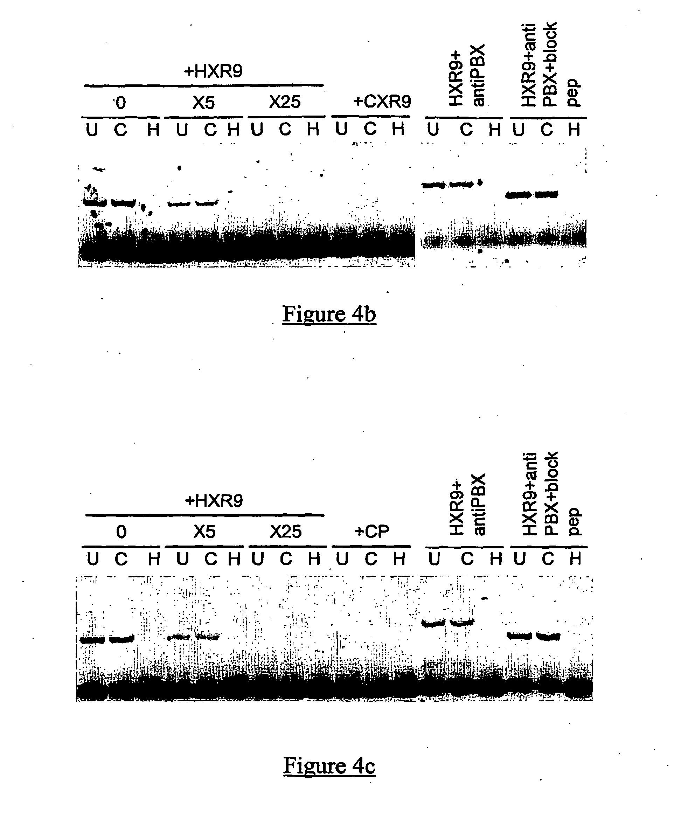

Figure 4 shows the specificity ofhexapeptide action.

Figure 4(a): HXArg9(2D) blocks the binding of HOXD9 to PBX. B16 murine melanoma cells were treated with 60µM HXArg9(2D) (HXR9) or CX2Arg9(2D) (CXR9) for 4 hours. Protein was then extracted from these cells using standard methods, and PBX proteins were precipitated using an anti-PBX antibody. Precipitates were probed for HOXD9, GR and PBX, as shown. HXArg9(2D) blocks the binding of PBX to HOXD9 but not to GR.

Figure 4(b): HXArg9(2D) prevents the formation of HOX/PBX/DNA complexes in cultured B16 cells: Double stranded oligodexoynucleotide probes were incubated in protein extracted from B16 cells. One of these probes (HP) contained a HOX/PBX consensus binding region (bold) - 5' CTGTTTGATT TATTTGTTTA 3'. A second, control probe (CP) contained an altered HOX/PBX binding site that is not predicted to bind HOX and PBX proteins - 5' CTGTTACTGA CGATTGTTTA 3'. Protein extracted from cells treated with the control CX2Arg9(2D) peptide caused a mobility shift in HP (lane 2) that could be abolished by 25 fold excess unlabelled HP (lane 8), and enhanced (super shifted) by anti-PBX antibody (lane 14). The mobility of CP was unchanged by incubating with the protein extract (lane 11). Protein extracted from HX treated cells did not change the mobility of HP (lane 3) or CP (lane 12), and the mobility of HP was also unchanged in response to unlabelled probe (lane 9) or anti-PBX antibody (lane 15). U, untreated; C, CX2Arg9(2D) treated; H, HXArg9(2D) treated; +HP, the HP probe was added to the lysates with an excess of unlabelled probe as shown (0, 5 fold, or 25 fold); +CP, control probe added to lysates; '+anti PBX', anti-PBX antibody added to lysate; '+block pep', a blocking peptide was added to the lysate that blocks anti-PBX antibody from binding to PBX.

Figure 4(c): HXArg9(2D) prevents the formation of HOX/PBX/DNA complexes in tumours. Double stranded oligodexoynucleotide probes were incubated in protein extracted from B16 tumours. One of these probes (HP) contained a HOX/PBX consensus binding region (bold) - 5' CTGTTTGATT TATTTGTTTA 3'. A second, control probe (CP) contained an altered HOX/PBX binding site that is not predicted to bind HOX and PBX proteins - 5' CTGTTACTGA CGATTGTTTA 3'. Protein extracted from tumours treated with the control CX2Arg9(2D) peptide caused a mobility shift in HP (lane 2) that could be abolished by 25 fold excess unlabelled HP (lane 8), and enhanced (super shifted) by anti-PBX antibody (lane 14). The mobility of CP was unchanged by incubating with the protein extract (lane 11). Protein extracted from HXArg9(2D) treated tumours did not change the mobility of HP (lane 3) or CP (lane 12), and the mobility of HP was also unchanged in response to unlabelled probe (lane 9) or anti-PBX antibody (lane 15). U, untreated; C, CX2Arg9(2D) treated; H, HXArg9(2D) treated; +HP, the HP probe was added to the lysates with an excess of unlabelled probe as shown (0, 5 fold, or 25 fold); +CP, control probe added to lysates; '+anti PBX', anti-PBX antibody added to lysate; '+block pep', a blocking peptide was added to the lysate that blocks anti-PBX antibody from binding to PBX.

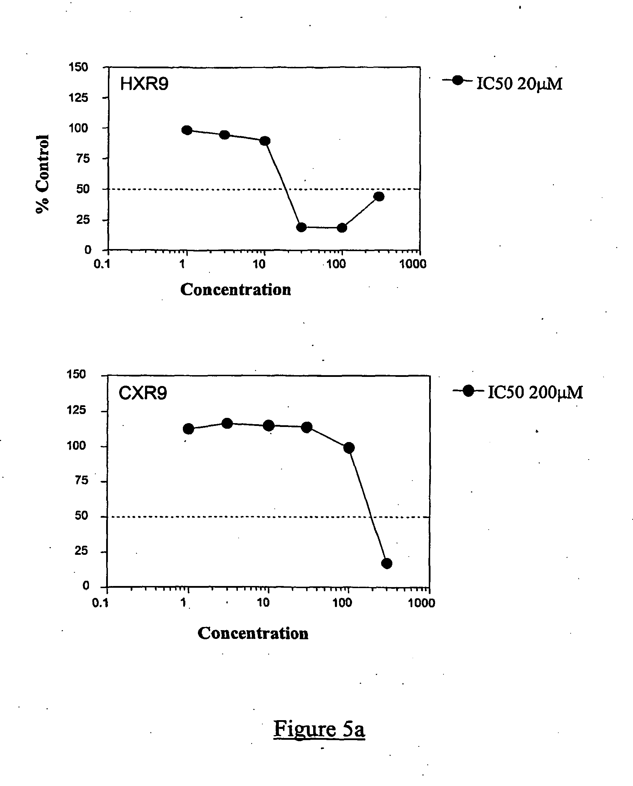

Figure 5(a): MTT assays to determine the IC50 of the control and hexapeptide-containing peptides.

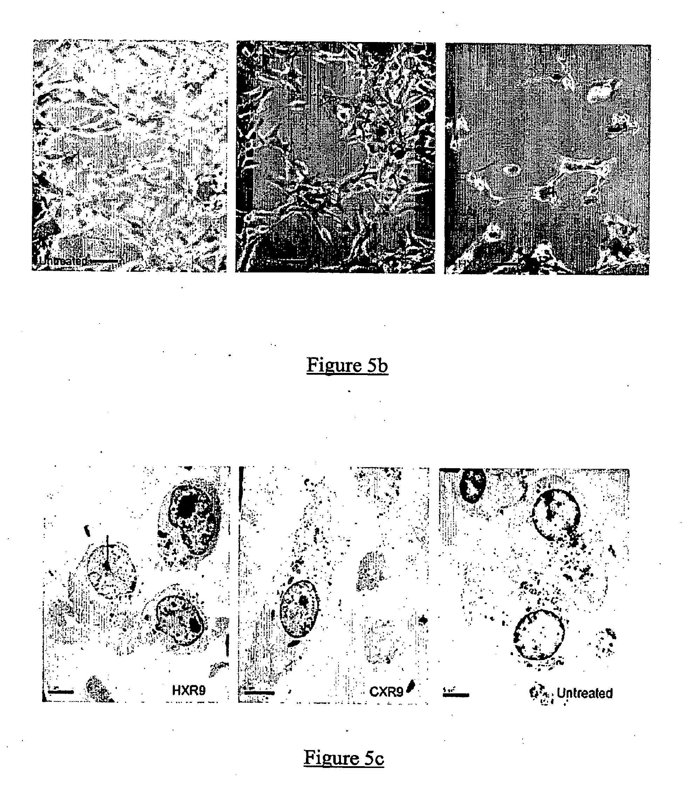

Figure 5(b): Light micrographs of B16 cells treated with 60µM HXArg9(2D) (HXR9) or CX2Arg9(2D) (CXR9) for two hours. Scale bar = 40µm.

Figure 5(c) Transmission electron micrographs of B 16 cells treated with 60µM HXR9 or CXR9 for two hours. Scale bar = 5µM.

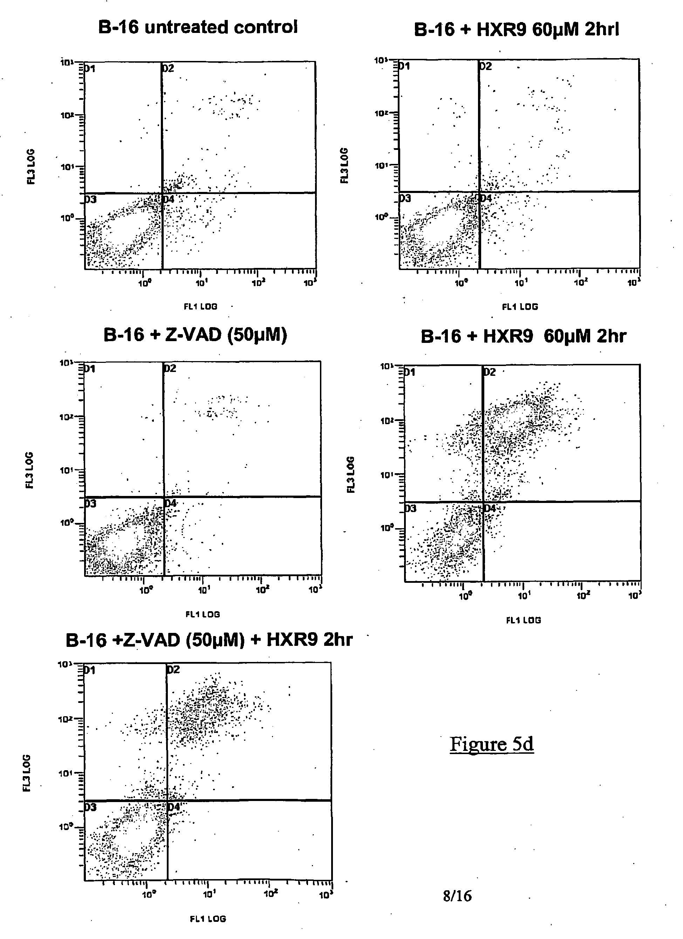

Figure 5(d) FACS sorting of B16 cells with Annexin. Cells were treated as indicated above each plot. The lower left hand quadrant represents normal, viable cells. The bottom and top right hand quadrants represent cells in early and late apoptosis, respectively, and the top left hand quadrant represents cells in necrosis. Z-VAD, an inhibitor of caspase-mediated apoptosis, causes a significant reduction in the number of cells undergoing apoptosis as a consequence of HXArg9(2D) treatment.

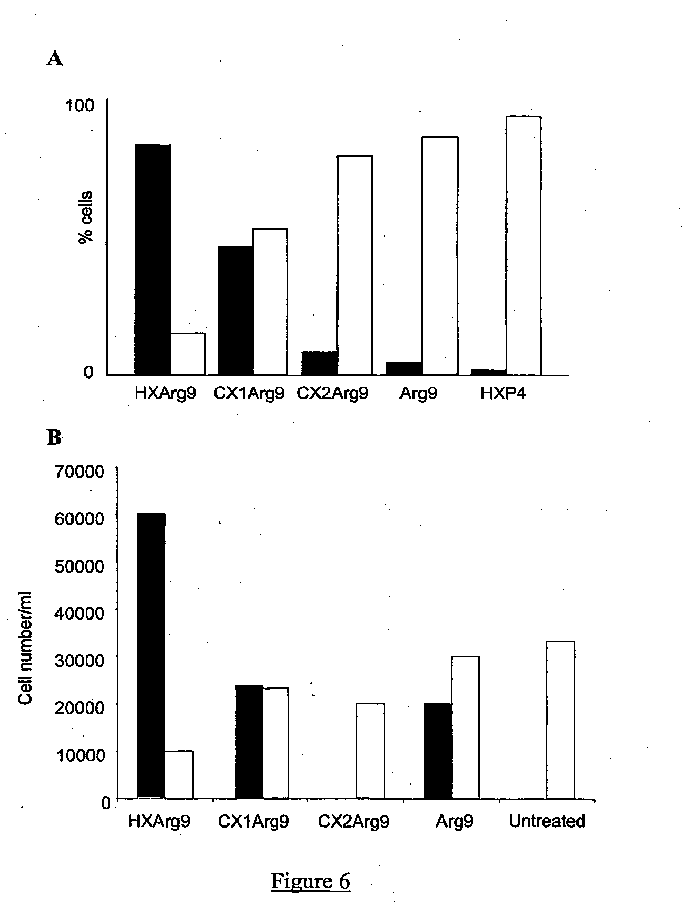

Figure 6 shows the effect of peptides on B16 murine melanoma cells. The peptides as indicated were applied to the cells at a concentration of 60µM (Figure 6A) or 6µM (Figure 6B). In both experiments the HXArg9, CX1Arg9 and CX2Arg9 peptides used were the (2D) variant peptides in which the N- and C- terminal amino acids are in the D conformation and all remaining amino acids are in the L conformation. The number of living and dead cells were assessed after 3 hours. Black bars = % or number of cells dead. White bars = % or number of cells alive.

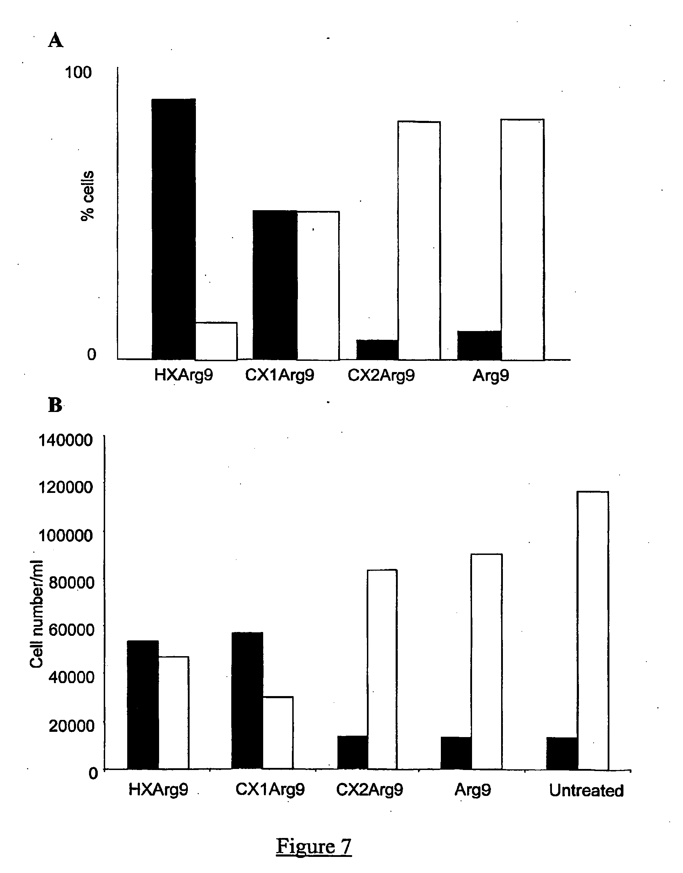

Figure 7 shows the effect of peptides on LNCAP human prostate cells. The peptides as indicated were applied to the cells at a concentration of 60µM (Figure 7A) or 6µM (Figure 7B). In both experiments the HXArg9, CX1Arg9 and CX2Arg9 peptides used were the (2D) variant peptides in which the N- and C- terminal amino acids are in the D conformation and all remaining amino acids are in the L conformation. The number of living and dead cells were assessed after 3 hours. Black bars = % or number of cells dead. White bars = % or number of cells alive.

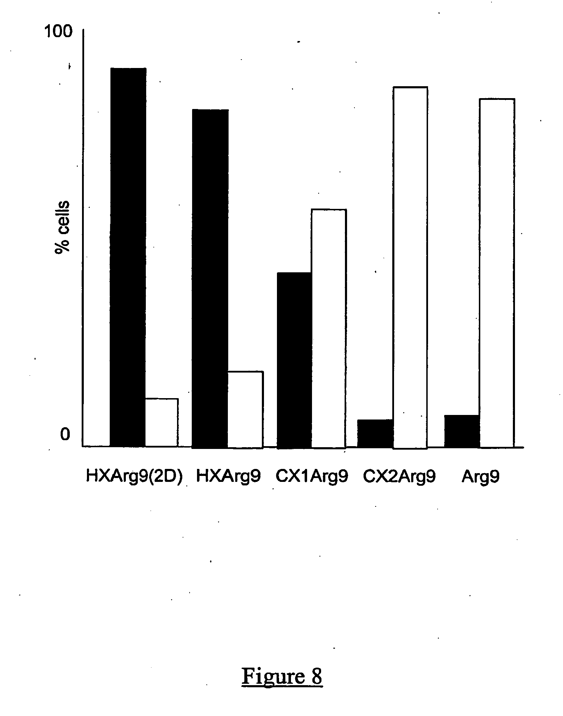

Figure 8 shows the effect of peptides on K562 human chronic myeloid leukaemia cells. The peptides as indicated were applied to the cells at a concentration of 60µM. HXArg9(2D) is the variant form of HXArg9 in which the N-terminal and C-terminal amino acids are in the D conformation and the remaining amino acids are in the L conformation. HXArg9 consists of only L-conformation amino acids. The CX1Arg9 and CX2Arg9 peptides used were the (2D) variant peptides in which the N- and C- terminal amino acids are in the D conformation and all remaining amino acids are in the L conformation. The number of living and dead cells were assessed after 3 hours. Black bars = % cells dead. White bars = % cells alive.

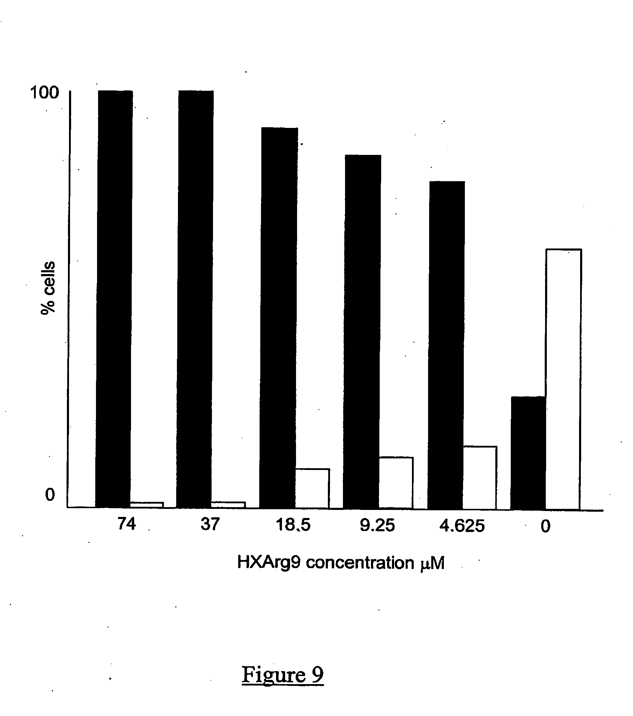

Figure 9 shows the effects of different doses of the HXArg9 peptide on B16 murine melanoma cells. The HXArg9 peptide used was the (2D) variant peptide in which the N- and C- terminal amino acids are in the D conformation and all remaining amino acids are in the L conformation. HXArg9 was applied to the cells at a variety of concentrations (µM), and the number of dead and living cells was assessed after 3 hours. Black bars = % cells dead. White bars = % cells alive.

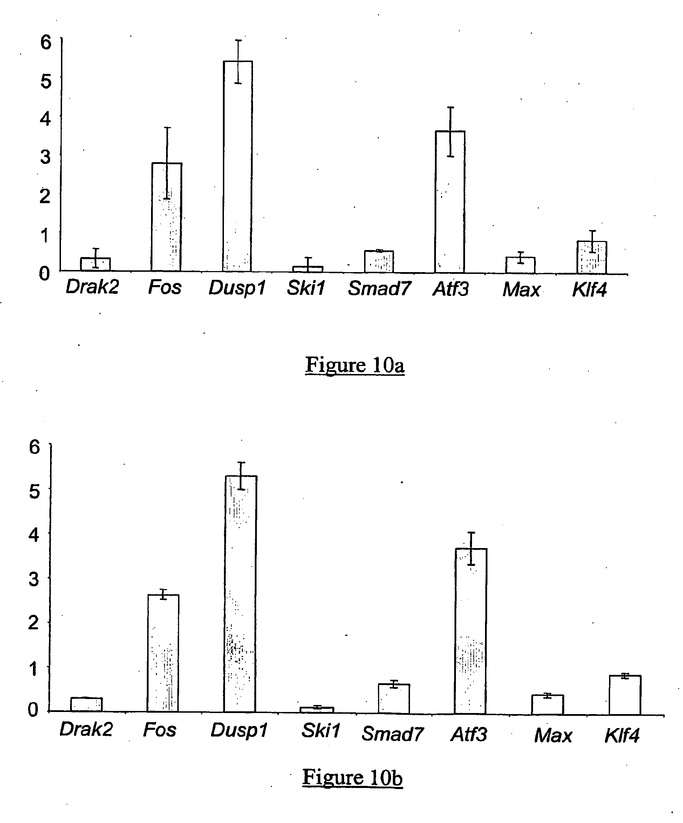

Figure 10. Semi-quantitative PCR of those genes identified by microarray as being upregulated in response to HXArg9(2D). RNA extracted from (a) B16 cells cultured in vitro and treated with 60µM peptide for two hours, and (b) B16 tumours treated with peptide injected IP at 15mg / Kg body weight; tumours were excised and RNA was extracted two hours after treatment. Results are expressed as a proportion of the level of β-actin transcripts. For (a), the results are an average of 2 separate experiments, for (b), the results are an average for tumours taken from three individual mice. U, untreated; C, CX2Arg9(2D) treated; H, HXArg9(2D) treated. Error bars represent the standard error of the mean.

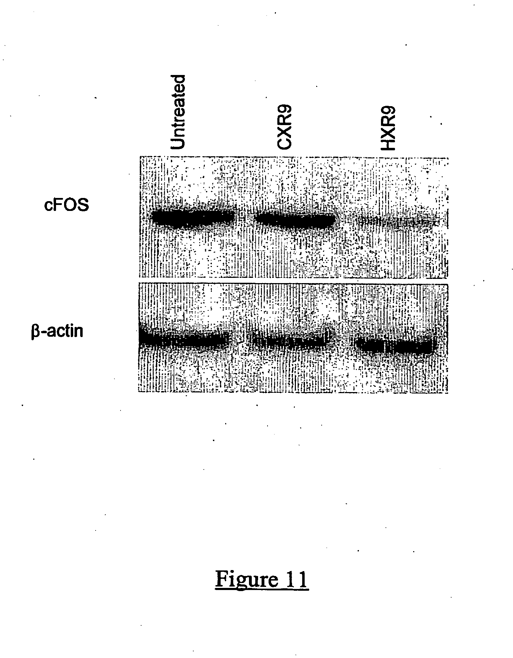

Figure 11. cFOS protein is greatly depleted after HXArg9(2D) treatment.
Protein was extracted from B16F10 cells treated with 60µM HXArg9(2D) (HXR9) or CX2Arg9(2D) (CXR9) for two hours. Western analysis using an anti-cFOS antibody reveals a significant reduction in the amount of cFOS protein after HXArg9(2D) treatment. Loading control: β-acting.

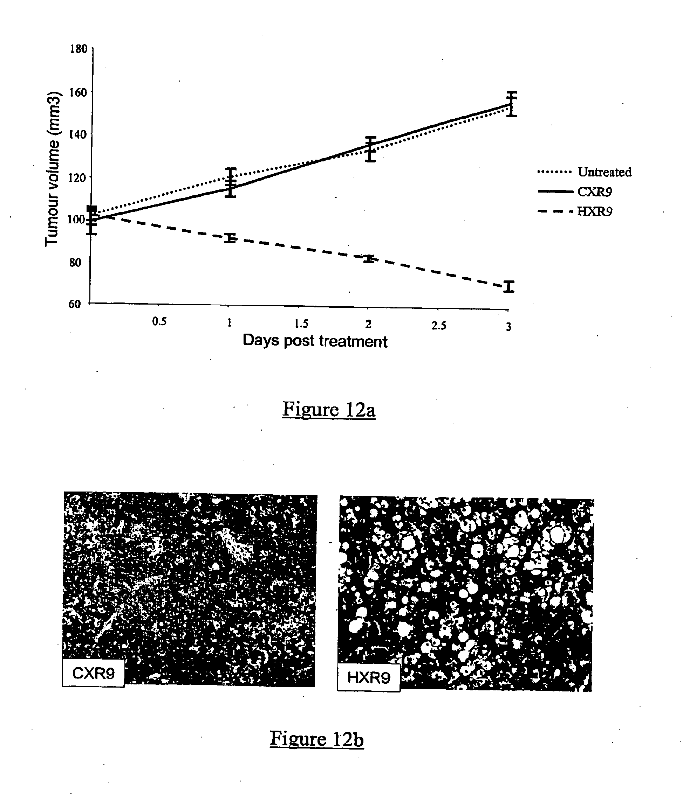

Figure 12. Treatment of murine B16 tumours in vivo. Mice were injected with one million B16 cells subcutaneously and treatment was started when tumours first became palpatable (day 1 = first day after treatment). Mice were injected once daily for four days with an intraperitoneal dose of 15 mg/kg. (a) Changes in tumour size over treatment period (n=4). (b) Histological sections of tumours from HXArg9(2D) (HXR9) and CX2Arg9(2D) (CXR9) treated mice at the end of treatment.

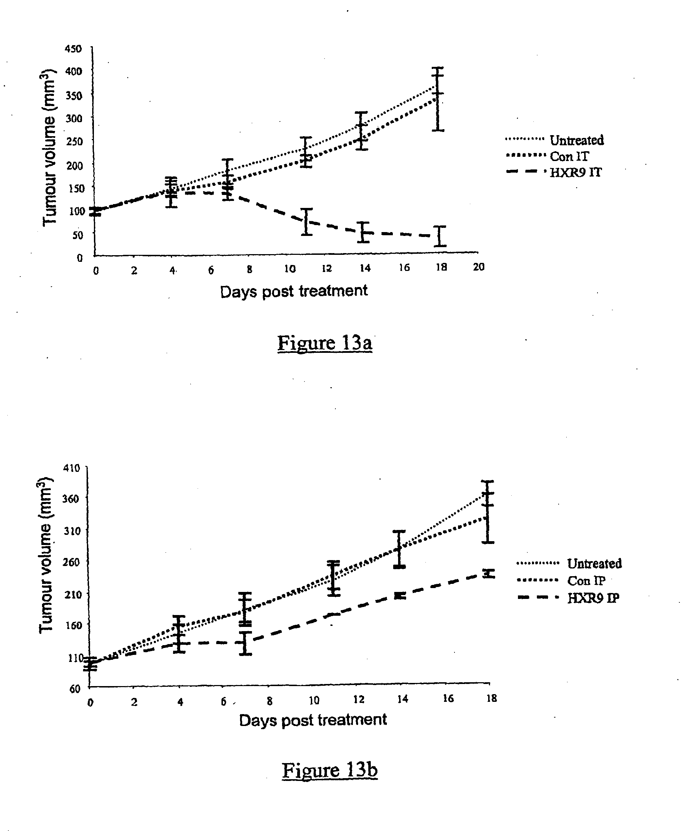

Figure 13. Treatment of A549 tumours *in vivo*. Nude mice were injected with one million A549 cells subcutaneously and treatment was started when tumours reached an average volume of 100mm³. Mice were treated twice weekly with HXArg9(2D) at 100mg/Kg for the first 4 treatments and then 10mg/kg for remaining treatments (n=4). (a) intralesional (IT) administration, (b) intraperitoneal (IP) administration.

### Detailed Description of the Invention

The present invention relates to molecular mimics of the HOX hexapeptide region. It is now shown that molecules which impair PBX-dependent transcription regulation (e.g. activation or repression), e.g. by interfering with the interaction between PBX and its co-factors, preferably HOX, and its target DNA, e.g. molecules which affect the binding of HOX and PBX proteins, have downstream effects which can offer great advantages such as preventing or reducing aberrant cell division and maintaining pluripotency of stem cells.

Preferred PBX modulators are peptides. "Peptides" as referred to herein are molecules with less than 100 amino acid residues, but are preferably shorter, e.g. less than 50, e.g. less than 30 residues in length, preferably from 10 to 25 residues in length.

The Inventors have found that by attaching such a PBX modulator peptide to a cationic polymer of basic amino acids, such as a polyarginine sequence, improved effectiveness can be achieved. In particular, by using such a cationic polymer as a cell penetration moiety, the effects of the peptide may be seen much more rapidly than when other cell penetration sequences are used. In one embodiment, peptides of the invention comprise or consist of the general formula (I) (SEQ ID NO: 1):

Y₁ X₁ X₂ X₃ W X₄ X₅ X₆ X₇ X₈ Y₂ (I)

or a functionally equivalent derivative thereof which may optionally be substituted, e.g. with a label or attachment moiety, wherein
- X₁ is the amino acid W;
- X₂ is the amino acid Y;
- X₃ is the amino acid P;
- X₄ is an amino acid, selected from M, L, I or V;
- X₅ is the amino acid K or R;
- X₆ is the amino acid K or R;
- X₇ is the amino acid H or T;
- X₈ is one or more amino acids or absent, for example X₈ may be H, T or absent; and
- Y₁ and Y₂ are each either absent or a peptide comprising the cationic polymer of basic amino acids (Arg)₉, such that at least one of Y₁ and Y₂ is present. In one embodiment, Y₁ and/or Y₂ acts as a cell penetration moiety or comprises a sequence which acts as a cell penetration moiety.

Generally, the X₁ to X₈ sequence corresponds to the sequence capable of interfering with the interaction between HOX and PBX *in vivo.* Generally, the Y₁ and/or Y₂ moiety acts as a cell penetration moiety to allow or assist the entry of the peptide into a cell. The peptide of the invention comprises or consists of any of the sequences X₁ to X₈ described herein, attached to any of the peptides Y₁ and/or Y₂ described herein. That is, the cationic polymer of basic amino acids described herein may be used in combination with any X₁ to X₈ sequence described herein, and may be located at the C-terminal, at the N-terminal, or at both termini of the peptide X₁ to X₈.

In the above sequence (I), X₁ to X₅ forms the hexapeptide sequence.

Preferred peptides of formula (I) have the formula:
Y₁ W Y P W M K K H H Y₂ (SEQ ID NO: 3)
or functionally equivalent derivatives, variants or fragments thereof, wherein Y₁ and Y₂ are as defined herein.

In one embodiment, said sequence X₁ to X₈ may be WYPWMKKHH or WYPWMKKHHR, or X₁ to X₈ may be a variant of said sequence WYPWMKKHH, for example a variant wherein one, two, three, four or more amino acids are varied within the constraints of formula (I) above.
For example, M at position X₄ may be replaced by L, I or V; K at position X₅ may be replaced by R; K at position X₆ may be replaced R; H at position X₇ may be replaced by T; H at position X₈ may be replaced by any other one or more amino acids or may be absent, for example X₈ may be T or absent. Any one, two, three, four or five of these substitutions may be carried out to create an alternative peptide falling within the scope of formula (I) above.

For example, in one embodiment, residues X₁ to X₈ in formula (I) above may be:
- X₁ = W; X₂ = Y; X₃ = P; X₄ = M/I/V/L; X₅ = K/R; X₆ = K/R; X₇ = H/T; X₈ = H/HR/T/absent
- X₁ = W; X₂ = Y; X₃ = P; X₄ = M/I/V/L; X₅ = K/R; X₆ = K/R; X₇ = H/T; X₈ = any amino acid or absent

Suitable sequences for X₁ to X₈ include:

| | | | |
|---|---|---|---|
| WYPWMKKHH | WYPW**V**KKHH | WYPW**I**KKHH | WYPWM**R**KHH |
| WYPWMK**R**HH | WYPWM**RR**HH | WYPWMKK**T**H | WYPWMKKH**T** |
| WYP WMKK**TT** | WYPWMK**RT**H | WYPWM**R**K**T**H | WYPWM**RR**TH |
| WYPWM**R**KH**T** | WYPWMK**RH**T | WYPWM**RR**HT | WYPWM**RRTT** |
| WYPW**LR**KHH | WYPW**L**K**R**HH | WYPWMKKH | |

Any of these variant X₁-X₈ sequences may be used in combination with the Y₁ and/or Y₂ residues described herein. For example, any of the X₁-X₈ sequences described herein may be used with a (Arg)₉ peptide attached at the C-terminal, at the N-terminal or at both ends.

Generally, Y₁ and/or Y₂ will comprise a sequence capable of acting as a cell penetration moiety.

Y₁ is preferably attached via the N-terminal amino group on X₁, but alternatively via a side chain of X₁, Y₂ is preferably attached via the C-terminal carboxyl group on X₈, but alternatively via a side chain of X₈.

As used herein a "cell penetration moiety" refers to a molecule, structure or collection of molecules which assist or facilitate entry of the molecule to which it is attached into a cell.

The cell penetration moiety may be directly linked to the peptide X₁ to X₈, or may be attached via a linker sequence of one or more amino acids. The linker sequence may comprise the amino acid(s) at position X₈. Typically, the linker comprises amino acids that do not have bulky side groups and therefore do not obstruct the folding of the protein such as serine and glycine. The linker permits the cell penetration moiety to assist or facilitate entry of the peptide into a cell and also allows the HOX-PBX interacting part of the peptide to interfere with HOX-PBX binding. The linker may be a flexible amino acid linker. The linker typically has a length of up to 20 amino acids, such as 5 to 18 or 10 to 16, preferably 15 amino acids.

The cell penetration moiety may alternatively be associated with a peptide X₁ to X₈, e.g. may encapsulate or form a complex with said peptide, e.g. by using liposomes for lipofection or polycations or cationic lipids. "Associated with" as used herein refers to the moiety being attached to, or connected in some way, to the peptide.

In one embodiment Y₁ and/or Y₂ is, or comprises, the cationic polymer (Arg)₉ or a physiologically acceptable salt thereof. Such pharmaceutically acceptable salts include for example, mineral acid salts such as hydrochlorides, hydrobromides, phosphates, sulphates and the like and salts of organic acids such as acetates, propionates, malonates, benzoates and the like. Typically the salt is a hydrochloride or a sulphate.

Y₁ and/or Y₂ is, or comprises a cationic polymer of the basic amino acid arginine. Such polyamino acidis are readily available from Sigma-Aldrich.

The amino acids in any of the above polyamino acids may be L or D amino acids.

The polyamine is a homopolymer of arginine. Exemplified herein are peptides wherein Y₁ and/or Y₂ is (Arg)₉, e.g. where Y₁ is absent and Y₂ is Arg-Arg-Arg-Arg-Arg-Arg-Arg-Arg-Arg (SEQ ID NO: 9). The Preferred peptides are those wherein Y₁ and/or Y₂ = (Arg)₉.

Particularly preferred peptides have the sequence
WYPWMKKHHRRRRRRRRR (SEQ ID NO: 6).

The amino acids in the peptides of formula (I) may be L or D amino acids. All the amino acids in the peptide may have the same stereochemistry, for example, the peptide may consist of only L-amino acids or only D-amino acids. Alternatively, the peptide may comprise a combination of both L- and D- amino acids. As explained below, by varying the number and position of L- and D- amino acids in the peptide, it may be possible to effect the stability of the resultant peptide, for example, the stability of the peptide after administration to the body. In one embodiment at least the N-terminal- and C-terminal-most amino acids of the peptide are in the D-conformation while the remaining amino acids are in the L- conformation.
Optionally one, two, three, four or more further amino acids are also in the D conformation. Optionally one or more amino acids at, or adjacent to position X₈ are in the D conformation. Optionally the amino acids at positions X₂ to X₄ are in the L conformation.

The Inventors have found that the stability of PBX modulator peptides may be improved by incorporating one or more D-amino acids in the peptide. In particular, the half life of peptides in plasma may be improved by using D amino acids in at least the N- and C-terminal positions of the peptide. This improved half life is seen in peptides which comprise a variety of cell penetration sequences.

A "cell penetration moiety" refers to a molecule, structure or collection of molecules which assist or facilitate entry of the molecule to which it is attached into a cell. A variety of such moieties are well-known in the art and include peptides such as penetratins, tat-derived proteins, peptide signal sequences that allow cell entry, peptides comprising such peptide signals as well as synthetic and/or chimeric cell-penetrating peptides such as transportan or model amphipathic peptides (Lindgren et al., 2000, TiPS, 21, p99-103 and Derossi et al., 1998, Trends C. Biol., 8, p84-87). Non-peptide molecules or substances which are capable of entering cells may also be used. Preferably said cell penetration moiety acts by a receptor-independent mechanism. Any substance that can allow or help a molecule, such as a peptide of the invention, to enter a cell may be used. The moiety may be a generally acting substance that can enter a variety of cell types, or may be specific or targeted to a particular cell type to be treated. '

As mentioned above, the peptide may be substituted, preferably at the N- or C- terminus, by a further moiety. Such moieties may be added to aid the function of the peptide, its targeting or its synthesis, capture or identification, e.g. a label (e.g. biotin) or lipid molecules. Such moieties may alternatively be found within the peptide itself. For example a moiety such as a label may be attached to an amino acid located internally within the peptide. For example, X₈ may comprise all or part of such a moiety, or said moiety may form part of, or be located within, Y₁, Y₂, Y₃ and/or Y₄.

Peptides of the invention, as defined herein, may be chemically modified, for example, post-translationally modified. For example they may be glycosylated or comprise modified amino acid residues. They can be in a variety of forms of polypeptide derivatives, including amides and conjugates with polypeptides.

Chemically modified peptides also include those having one or more residues chemically derivatized by reaction of a functional side group. Such derivatized side groups include those which have been derivatized to form amine hydrochlorides, p-toluene sulfonyl groups, carbobenzoxy groups, t-butyloxycarbonyl groups, chloroacetyl groups and formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine.

Also included as chemically modified peptides are those which contain one or more naturally occurring amino acid derivatives of the twenty standard amino acids. For example, 4-hydroxyproline may be substituted for proline or homoserine may be substituted for serine.

A peptide of the invention may carry a revealing label. Suitable labels include radioisotopes such as ¹²⁵I, ³²P or ³⁵S, fluorescent labels, enzyme labels, or other protein labels such as biotin.

Peptides as described above for use in accordance with the invention may be prepared by conventional modes of synthesis including genetic or chemical means. Synthetic techniques, such as a solid-phase Merrifield-type synthesis, may be preferred for reasons of purity, antigenic specificity, freedom from unwanted side products and ease of production. Suitable techniques for solid-phase peptide synthesis are well known to those skilled in the art (see for example, Merrifield et al., 1969, Adv. Enzymol 32, 221-96 and Fields et al., 1990, Int. J. Peptide Protein Res, 35, 161-214). Chemical synthesis may be performed by methods well known in the art involving cyclic sets of reactions of selective deprotection of the functional groups of a terminal amino acid and coupling of selectively protected amino acid residues, followed finally by complete deprotection of all functional groups.

Synthesis may be performed in solution or on a solid support using suitable solid phases known in the art.

In an alternative embodiment a peptide of the invention may be produced from or delivered in the form of a polynucleotide which encodes, and is capable of expressing, it. Such polynucleotides can be synthesised according to methods well known in the art, as described by way of example in Sambrook et al (1989, Molecular Cloning - a laboratory manual; Cold Spring Harbor Press). Such polynucleotides may be used *in vitro* or *in vivo* in the production of a peptide of the invention. Such polynucleotides may therefore be administered or used in the manufacture of a medicament for the treatment of cancer or another disease or condition as described herein.

The present invention also includes expression vectors that comprise such polynucleotide sequences. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for expression of a peptide of the invention. Other suitable vectors would be apparent to persons skilled in the art. By way of further example in this regard we refer to Sambrook *et al.*

Thus, the peptide may be provided by delivering such a vector to a cell and allowing transcription from the vector to occur. Preferably, a polynucleotide of the invention or for use in the invention in a vector is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

The vectors may be for example, plasmid, virus or phage vectors provided with an origin of replication, optionally a promoter for the expression of the said polynucleotide and optionally a regulator of the promoter. The vectors may contain one or more selectable marker genes, for example an ampicillin resistence gene in the case of a bacterial plasmid or a resistance gene for a fungal vector. Vectors may be used *in vitro,* for example for the production of DNA or RNA or used to transfect or transform a host cell, for example, a mammalian host cell. The vectors may also be adapted to be used *in vivo,* for example to allow *in vivo* expression of the polypeptide.

Promoters and other expression regulation signals may be selected to be compatible with the host cell for which expression is designed. For example, yeast promoters include *S. cerevisiae* GAL4 and ADH promoters, *S*. *pombe nmt*1 and *adh* promoter. Mammalian promoters, such as b-actin promoters, may be used. Tissue-specific promoters are especially preferred. Mammalian promoters include the metallothionein promoter which can be induced in response to heavy metals such as cadmium. Viral promoters may also be used, for example the SV40 large T antigen promoter, adenovirus promoters, the Moloney murine leukaemia virus long terminal repeat (MMLV LTR), the rous sarcoma virus (RSV) LTR promoter, the SV40 promoter, the human cytomegalovirus (CMV) IE promoter, adenovirus, HSV promoters (such as the HSV IE promoters), or HPV promoters, particularly the HPV upstream regulatory region (URR). All these promoters are readily available in the art.

The invention also includes cells that have been modified to express a peptide of the invention. Such cells include transient, or preferably stable higher eukaryotic cell lines, such as mammalian cells or insect cells, lower eukaryotic cells, such as yeast or prokaryotic cells such as bacterial cells. Particular examples of cells which may be modified by insertion of vectors encoding for a peptide of the invention include mammalian HEK293T, CHO, HeLa and COS cells. Preferably the cell line selected will be one which is not only stable, but also allows for mature glycosylation and cell surface expression of a polypeptide. Expression may be achieved in transformed oocytes. A suitable peptide may be expressed in cells of a transgenic non-human animal, preferably a mouse. A transgenic non-human animal expressing a peptide of the invention is included within the scope of the invention. A peptide of the invention may also be expressed in *Xenopus laevis* oocytes or melanophores.

The present invention also extends to antibodies (monoclonal or polyclonal) and their antigen-binding fragments (e.g. F(ab)2, Fab and Fv fragments i.e. fragments of the "variable" region of the antibody, which comprises the antigen binding site) directed to peptides as defined hereinbefore, i.e. which bind to epitopes present on the peptides and thus bind selectively and specifically to such peptides, and which may be used in the methods of the invention.

The peptides of the invention, as described above, are able to specifically block the interaction between PBX and HOX. As shown in the Examples, peptides of the invention have been found to ablate or reduce the proliferation of a range of cancer cell types. Accompanying these changes, down-regulation of a number of known HOX targets may be observed. As described in more detail below, the peptides of the invention may therefore have therapeutic uses in the treatment of cancers in which Hox genes are expressed, as cytoprotective agents during other cancer therapies or in the *ex vivo* protection of stem cell cultures.

Peptides described above may be used to block interactions of PBX with its binding partners, e.g. HOX, and preferably thereby prevent the binding of HOX to its target DNA. Thus in a further aspect the present invention provides use of a peptide as described herein to reduce or inhibit binding of PBX to a binding partner, preferably HOX, or the use of such peptides to reduce or inhibit binding of HOX to its target DNA.

"PBX" refers to the family of pre-B-cell transformation related genes and includes genes encoding extradenticle homeoprotein proteins and homologues of the Drosophila extradenticle gene, such as genes in vertebrates. Vertebrate PBX proteins are transcription factors that contain a homeodomain (Mann et al., 1996).

"HOX" refers to homeobox genes which contain a sequence which encodes a homeodomain of about 60 amino acids and a sequence which encodes the hexapeptide sequence N-terminal to the homeodomain (Morgan et al., 2000, TIG, 16(2), p66-67 and Kru.mlauf, 1994, Cell, 78(2), p191-201). The HOX proteins are transcription factors that act to define anterior-posterior development in early development. Such PBX or HOX genes or proteins as described herein include homologues present in any multicellular animal, but are preferably from vertebrates, e.g. from mammals, especially preferably from humans.

As referred to herein "binding" refers to the interaction or association of at least two moieties iri a reversible or irreversible reaction, wherein said binding is preferably specific and selective.

As used herein a "binding partner" refers to a molecule which recognizes and binds specifically (i.e. in preference to binding to other molecules) to its binding partner. Such binding pairs when bound together form a complex.

A "reduction in binding" refers to a decrease in binding, e.g. as manifest by an increased concentration of one of the binding pair required to achieve binding. Reduction includes a slight decrease as well as absolute abrogation of specific binding. A total reduction of specific binding is considered to equate to a prevention of binding. "Inhibition" refers to competitive interference of the binding of the binding partners by the peptide, which serves to reduce the partners' binding. Agents which prevent or reduce PBX-dependent transcription regulation, have been found to have advantageous effects on aberrant cell division as described herein. Such agents are preferably those which prevent, reduce or inhibit the binding of PBX to its binding partners, especially preferably the binding between PBX and HOX . (such as antagonists of the interaction between HOX and PBX, e.g. the peptides described hereinbefore). However, suitable agents also include those that affect binding of the transcription factors to the target DNA, e.g. which block the interaction of PBX or its binding partner, such as HOX, to the target DNA. Especially preferably such agents prevent HOX-dependent transcription regulation.

Whilst not wishing to be bound by theory, it is believed that antagonists of HOX:PBX binding prevent the interaction between multiple important HOX:PBX protein binding partners, and the HOX proteins are therefore unable to act as transcription factors on the genes to which they bind. The failure to regulate expression of these genes may have numerous effects on the cells, for example reducing or preventing the excessive cell division. Similarly any moiety which prevents or reduces PBX-dependent transcription regulation, e.g. blocks the interaction of HOX with its target DNA, may be expected to have similar effects.

In a further aspect, therefore, the present invention can be used for reducing aberrant cell division wherein said cells are administered an agent which prevents or reduces PBX-dependent transcription regulation, preferably which reduces or prevents binding of PBX to a binding partner, preferably to HOX (preferably HOXB4, HOXB8 or HOXA9) or reduces or prevents binding of HOX to its target DNA, preferably an antagonist, especially preferably an antagonist of the interaction between HOX and PBX, especially preferably a peptide as described herein (and which preferably inhibits HOX-dependent transcription regulation).

Agents which are suitable for this purpose include antagonists of the interaction between HOX and/or PBX and the DNA target to which they bind, antagonists of the interaction between PBX and its binding partners especially HOX proteins, or agents which impair the binding ability of HOXlPBX or the target DNA, e.g. which block relevant sites or cause structural changes at relevant sites on HOX/PBX or the target DNA or reduce the number of molecules available for binding (which may be achieved by for example modifying the expression/expressed product of PBX/HOX). Preferably however, antagonists are employed.. Preferred agents are the peptides of the invention as described above.

As described herein, "aberrant cell division" refers to cell division above the normal level (i.e. abnormal cell division) considered appropriate under the conditions which exist. Markers of aberrant cell division are well known to the person skilled in the art and can be used to determine whether a particular cell has been effected. For example, cells undergoing aberrant cell division may show atypical cytology, for example cellular pleomophism, nuclear pleomorphism, nuclear hyperchromatism or an increased nuclear:cytoplasmic ratio. Cells undergoing aberrant cell division may show a failure of cell differentiation. More particularly, such aberrant cell division may be present in certain conditions or diseases/disorders as described hereinafter, such as cancer.

"Reducing" cell division refers to reducing the rate of cell growth. Preferably cell growth is reduced to less than 0.5, especially preferably less than 0.25, e.g. less than 0.1 relative to control growth (without the agent) over the same time period (wherein control growth = 1). In a preferred aspect reduced cell division encompasses cell death/lack of viability which may occur in addition, or as an alternative to the reduction in cell growth. When cell death occurs preferably more than 50% of the existing cells, preferably more than 75% of the cells are destroyed.

As shown in the Examples, by adjusting the dose of the agent used it may also be possible to completely ablate some malignancies. Peptides of the invention may therefore be used to slow the growth of, or completely destroy, cancerous cells. As explained in more detail below; a suitable dose will depend on a number of factors and can be determined by a skilled practitioner.

As described herein "PBX-dependent transcription regulation" refers to activation or suppression of the transcription of genes by processes in which PBX plays a pivotal role, e.g. acts as a cofactor in the transcription regulatory complexes. Prevention or reduction refers to a measurable change in the extent of transcription. Prevention equates to a reduction in transcription to undetectable levels.

"Target DNA" refers to the gene containing the regulatory region to which PBX, HOX or any member of the transcription regulation complex containing such proteins, binds. As referred to herein, an "antagonist" is a molecule or complex of molecules which by virtue of structural similarity to one molecule of a binding pair competes with that molecule for binding to the other molecule of the binding pair. Antagonists for use in the invention include antagonists of the interaction between HOX and PBX which prevent or reduce binding between those entities. Preferred antagonists are peptides, antibodies, or anti-idiotypes in which these molecules bind to, or compete with the binding site on HOX or PBX. Preferably the antagonists compete by mimicking the PBX binding site on HOX, i.e. binding to PBX, e.g. peptides as described hereinbefore.

Other antagonists include those which prevent or reduce binding between HOX and its target DNA. HOX proteins are known to bind to a 6 base pair consensus sequence NNATTA on their target DNA and antagonists of this binding, e.g. oligonucleotides which are complementary to that sequence (e.g. sets of oligonucleotides with variability at 2 bases to accommodate the variability in the consensus sequence described above) are suitable as agents for the above described purpose.

Such methods may be performed *in vitro, in vivo* or ex vivo. Conveniently such methods are performed *in vivo* by the administration of said agent, preferably an antagonist as described hereinbefore, to a human or non-human animal to treat or prevent a.condition or disorder in which aberrant cell division occurs, e.g. cancer or a non-cancerous growth such as myelodysplastic syndrome (MDS). Alternatively expressed, the present invention provides the use of an agent as described hereinbefore, preferably an antagonist, especially preferably an antagonist of the interaction between HOX and PBX, e.g. a peptide as described hereinbefore, in the manufacture of a medicament for the treatment or prevention of a condition or disorder in which aberrant cell division occurs.

As referred to herein a "disorder" or a "disease" refers to an underlying pathological disturbance in a symptomatic or asymptomatic organism relative to a normal organism, which may result, for example, from infection or an acquired or congenital genetic imperfection.

A "condition" refers to a state of the mind or body of an organism which has not occurred through disease, e.g. the presence of a moiety in the body such as a toxin, drug or pollutant.

As defined herein "treatment" refers to reducing, alleviating or eliminating one or more symptoms of the condition or disorder which is being treated, relative to the symptoms prior to treatment. Treatment encompasses improving the condition of a patient having or suffering from the condition or disorder to be treated. For example, symptoms which may be affected include tumour size or numbers of cancerous cells in a given sample (or reduced stem cell numbers as described hereinafter).

"Prevention" of a condition or disorder refers to delaying or preventing the onset of a condition or disorder or reducing its severity, as assessed by the appearance or extent of one or more symptoms of said condition or disorder.

As an alternative to performing the methods *in vivo*, such methods may be performed *in vitro,* e.g. to reduce the cell division of, or eliminate, cells undergoing aberrant cell growth, in a sample. Appropriate culture conditions are as described for other methods of the invention as described hereinafter.

This is particularly useful in cell samples containing both normal and aberrant cells in which aberrant cells may be controlled/removed and the sample containing the normal cells used for subsequent procedures, e.g. returned to the donor body. This may be useful to, for example, eliminate aberrant haematopoietic blood cells from a blood sample of a patient, e.g. leukaemic cells, and the remaining cells may then be returned to the body of that patient.

Thus in a yet further aspect the present invention can be used for reducing aberrant cell division (preferably of reducing the growth, preferably involving the death and hence reducing the number, of cancer cells) in cells in a sample, wherein an agent as described hereinbefore is administered to said sample. For treating patients suffering from a disorder or condition typified by aberrant cell division (or preventing the same), said sample may be harvested from said patient and then returned to that patient as described hereinafter. In this context, a "sample" refers to any material obtained from a human or non-human animal, including embryonic, foetal, immature and adult stages of said animal, which contains cells undergoing aberrant cell division and include tissues and body fluids. "Body fluids" in this case include in particular blood, spinal fluid and lymph and "tissues" include tissue obtained by surgery or other means.

Preferably the aberrant cell division occurs in cells from eukaryotic organisms which may be any eukaryotic organisms such as human beings, other mammals and animals, birds, insects and fish.

Preferred non-human animals from which cells may be derived or on which methods of the invention may be conducted include, but are not limited to mammals, particularly primates, domestic animals, livestock and laboratory animals. Thus preferred animals include mice, rats, chickens, frogs, guinea pigs, cats, dogs, pigs, cows, goats, sheep, horses. Particularly preferably the cells are derived from, and the methods used to treat, or be prophylactic in, humans.

Preferably the cells undergoing aberrant cell division are cancer cells and the disorder to be treated or prevented is a cancer. Cancers that can be treated in this way are those cancers which involve the expression of HOX genes, wherein this HOX expression is reduced by the activity of a peptide of the invention, thus blocking the growth of, reducing the proliferation of, or leading directly to the death of, the cancerous cells. In a further embodiment, the peptide of the invention may act on the cancerous cells to move them from a quiescent state into the cell cycle and thus make them more susceptible to other, e.g. cytotoxic, anti-cancer treatments.

Preferably said cell to be treated expresses one or more Hox genes. For example, said cell may express one or more of HOXA1, HOXA3, HOXA4, HOXA5, HOXA7, HOXA9, HOXA11, HOXA13, HOXB1, HOXB2, HOXB3, HOXB4, HOXB8, HOXB9, HOXB13, HOXC4, HOXC6, HOXC8, HOXC10, HOXD3, HOXD4, HOXD8, HOXD9, HOXD10 and HOXD13. Said cell may express one or more of HOXB4, HOXB8 and HOXA9. It is possible that the level of Hox gene expression in the cell may be directly related to the sensitivity of the cell to the peptides of the invention. The peptides of the invention would therefore be more effective at treating cells which show high levels of HOX gene expression, for example higher levels of HOX gene expression than that in the surrounding tissue or higher levels of HOX gene expression than that of other cancer types where the cell is a cancer cell. The methods of the invention may therefore be particularly suitable where the cells to be treated show such increased or higher levels of HOX gene expression.

Preferably said cancers are malignant or pre-malignant or benign tumours and include carcinomas, sarcomas, gliomas, melanomas and lymphomas, including cancers of the bladder, kidney, pancreas, brain, head and neck, breast, gut, prostate, lung and ovary and leukaemias and lymphomas. Particularly preferred are colorectal, pancreatic, bladder, prostate, cervical, ovarian, gastric and non-small cell lung cancers.

Thus in a preferred aspect, the present invention can be used for treating or preventing cancer, in a human or non-human animal wherein said animal is administered an agent, preferably an antagonist, as described hereinbefore.

In some cancers, for example some forms of human pre-B cell leukaemia, PBX may act as an oncogene. The effects of PBX in such cancers will be different to that in other cancer types where PBX is not an oncogene. The effects of a peptide mimic of the invention may also therefore be different. In one embodiment, therefore, the present invention does not apply to such cancers because the effect of a peptide of the invention will be via a different mechanism to the PBX:HOX effect elucidated by the inventors. In this embodiment, therefore, a peptide of the invention may be used in the treatment or prevention of a cancer or other disorder in which aberrant cell division occurs, and in which PBX does not act as an oncogene. Preferably said cancerous cells express one or more Hox genes.For example, a suitable cancer for treatment by a method of the invention may be a leukaemia other than human pre-B cell leukaemia.

In some cancers, such as acute myeloid leukaemia (AML), peptides of the invention may block the proliferation of the cancerous cells, but may also stimulate those cells to leave the G0/G1 quiescent state and enter the cell cycle. These two effects are seen in the same cells under the same conditions. This is likely to be due to the cells being triggered to leave G0/G1 by the peptide (i.e. enter the cell cycle) but then failing to divide and instead either differentiating or undergoing apoptosis. The peptides of the invention may be used in the treatment of both primary AML and mature myeloid leukaemias. This suggests a specific utility for the peptides of the invention in acute myeloid and lymphoid leukaemias. Blocking PBX/HOX interactions in these cells using a peptide of the invention may therefore form an effective treatment for preventing leukaemia cell growth *in vivo.* In addition, by increasing the proportion of leukaemic cells that enter the cell cycle, the peptides of the invention may also increase their sensitivity to other cancer treatments such as chemotherapy. The peptides of the invention may therefore be used in combination with another cancer treatment as described further below.

Agents which prevent or reduce PBX-dependent transcription regulation have also been found to have beneficial effects on stem cells.

"Stem cells" as referred to herein are undifferentiated cells which are capable of differentiating into various cells, e.g. various blood cell types, and include haematopoietic (e.g. found in the bone marrow) and neural and hepatic stem cells, embryonic stem cells and embryonic germ cells and encompass both pluri- and toti-potent cells. Embryonic cells are considered to be those cells derived from the inner cell mass of the blastocyst and embryonic germ cells are those cells isolated from the primordial germ cell of the gonadal ridge of the 5 to 10 week old foetus. Preferably said cells are derived from eukaryotic organisms as described previously.

Prevention of PBX-mediated transcription regulation results in reduced, but continued, cell division and the appearance of molecular markers of differentiation (e.g. CD38). However on removal of the agent blocking that transcriptional regulation cells reverted to stem cells as assessed by the appearance of molecular markers (e.g. HOXB4, HOXB8, HOXA9, AC133), thus reflecting pluripotency of the cells. Whilst not wishing to be bound by theory, it is believed that despite the appearance of markers of differentiation/maturation, no phenotypic changes symptomatic of differentiation occur and the cells instead have a significantly reduced rate of cell cycling while the agent is being administered. On removal of the agent, the cells revert to stem cells.

It is also believed that treatment of pluripotent haematopoietic stem and progenitor cells (HSPCs) with a peptide of the invention blocks their proliferation, and increases the proportion of cells in the G0-G1 phase of the cell cycle. The longevity of the cultures confirms the effects of putative stem cells as well as more differentiated progenitor populations. The specificity of this inhibitory effect of peptides of the invention on these gene targets is underlined by its reversibility, with gene transcription and cell growth resuming on removal of the peptide.

These results have a number of applications which include maintenance or expansion of stem cells (e.g. in culture), for example for temporary storage of said cells, with possible expansion during that storage period. Such cells may then, for example, be used in clinical applications in which the addition of stem cells is desirable, e.g. to patients that have reduced numbers of stem cells and/or the ability to produce certain differentiated cell types, due to, for example, age, disease (e.g. cancers or autoimmune disease), congenital factors, environmental influences or contaminants and/or administered chemicals. In particular stem cells may be harvested from a patient prior to chemotherapy or radiotherapy and maintained and/or expanded and returned to that patient after chemotherapy or radiotherapy.

As an alternative example the stem cells may be used to provide cells from which a particular differentiated cell may be formed, e.g. neuronal cells, particularly in adult recipients where such suitable stem cells are absent or only low levels are present. The recipient of the stem cells is preferably also the donor, but may also be a different individual. The peptides of the invention may therefore be used to protect explanted tissue that contains stem cells (e.g. bone marrow cells) during culture *in vitro* or *ex vivo.*

Cells may also be maintained *ex vivo* or *in vivo,* for example to maintain viability during treatment that might normally affect their viability, e.g. during chemo- or radio-therapy. Agents as described herein, e.g. peptides of the invention can be used to reduce the susceptibility of stem cells to damage by such treatments by temporarily stopping or slowing the cell cycle of the stem cells. For example, peptides of the invention may be used to reduce the side effects caused by other cancer treatments, e.g. cytotoxic shock associated with many chemotherapeutic regimes. The cytoprotective effect of peptides of the invention on stem cells *in vivo* may also allow higher levels or doses of such cancer treatments to be used due to the decreased side-effects produced. For example, a higher dose of chemo-or radio-therapy may be possible.

Thus in a further aspect, the present invention provides a method of maintaining or expanding stem cells, wherein said method comprises at least the step of contacting said cells with an agent as described hereinbefore, preferably an antagonist, especially preferably an antagonist of the interaction between HOX and PBX, e.g. a peptide as described hereinbefore. This method may be used to maintain pluri- or toti-potency of the stem cells.

Preferably this method is performed *in vitro* or *ex vivo,* in culture, in which case the method may contain an initial step of harvesting stem cells from a donor. However, the method may also be used *in vivo* to maintain or improve the numbers of stem cells in an individual, particularly during exposure to agents or treatments that might cause stem cell damage. In such circumstances, the present invention can be used for maintaining or expanding stems cells in a patient wherein said patient is to be administered an agent as described hereinbefore, preferably an antagonist, especially preferably an antagonist of the interaction between HOX and PBX, e.g. a peptide as described hereinbefore.

"Maintaining" the cells refers to maintaining the viability of a large proportion of the starting, e.g. harvested, cells with minimal cell division, during the course of the treatment or culture period.

"Expanding" the cells refers to at least some cell division, preferably significant cell division, to increase the numbers of cells during the course of treatment, or culture.

As referred to herein "culture" refers to the growth or maintenance of the cells in a controlled artificial environment, i.e. *ex vivo.* Standard techniques for culture of cells are well known. Preferably cells are cultured at 37°C, 5% CO₂ in a humidified atmosphere in a standard culture medium. Preferably said culture is conducted for at least 2 hours, preferably more than 24 hours; e.g. between 24 hours and 8 weeks.

"Contacting" as used herein refers to any suitable technique which allows the agent to have access, and thus the possibility of binding, to cells in the sample, e.g. by application to the culture medium.

After the cells have been maintained or expanded, the agent may be removed to recover pluri- or toti- potency. When the method is performed *in vivo* this may be achieved by ceasing administration and allowing the body to clear the agent. *In vitro* or *ex vivo,* the agent is removed from the culture medium, e.g. by washing and replacement with fresh medium. Alternatively, the agent may be removed by allowing it to degrade naturally.

Thus the invention provides a method of maintaining or expanding stem cells and or obtaining pluri- or toti-potent stem cells, in culture, preferably an expanded population of said cells, wherein said method comprises at least the steps of:
a) contacting said cells in culture with an agent which reduces or prevents PBX-dependent transcription regulation as described hereinbefore, preferably an antagonist, especially preferably an antagonist of the interaction between HOX and PBX, e.g. a peptide as described hereinbefore; .
b) culturing said cells in the absence of said agent. It should be noted that the peptide becomes degraded within a few days during culture and thus active peptide is depleted. Thus, step b) may be performed without any prior washing if sufficient time has lapsed for degradation to occur. As mentioned previously, appropriate culture times are at least 2 hours, preferably more than 24 hours, e.g. between 24 hours and 8 weeks.

The method may use in an initial step stem cells harvested from a donor. Cells obtained by this and other methods of the invention comprise further aspects of the invention as does their use as a medicament.

The cells thus prepared by the above described *in vitro* or *ex vivo* methods may then be administered to an individual in need of such stem cells. Optionally, the cells may be modified prior to transplant, e.g. during the course of culturing or just prior to transplanting, e.g. by genetic modification, e.g. for gene transfer or to import a function not previously present in said cells, e.g. to compensate for a genetic deficit, e.g. by providing a missing factor, e.g. adenosine deaminase (ADA).

Thus in a yet further aspect, the present invention can be used for treating an individual in need of stem cells wherein stem cells prepared according to the above described method are administered to said individual.

Preferably said individual in need of said stem cells is an individual who has (or will have) lower than normal or desirable levels of such cells, which condition may exist normally, e.g. through age or as a result of external factors e.g. through chemotherapy or radiotherapy. Especially preferably, said stem cells are derived from the recipient individual.

Thus in a preferred feature the present invention can be used for improving the number of stem cells in a recipient individual by at least the steps of:
a) harvesting stem cells from a donor,
b) culturing said stem cells according to the methods described hereinbefore;
c) administering said cultured stem cells to said recipient individual.

Preferably said method is used for improving the number of stem cells in a patient subject to chemotherapy or radiotherapy, by at least the steps of:
a) harvesting stem cells from said patient prior to chemotherapy or radiotherapy,
b) culturing said stem cells according to the methods described hereinbefore;
c) administering said cultured stem cells to said patient after completion of chemotherapy or radiotherapy.

Alternatively described, harvesting step a) in the methods above may be absent and step b) may comprise culturing stem cells harvested from the donor according to the methods described hereinbefore. Said cells may be harvested by obtaining a sample of cells, tissue or body fluid from said donor and optionally extracting the cells therefrom.

As used herein a "sample" refers to any material obtained from the donor, e.g. human or non-human animal, including embryonic, foetal, immature and adult stages of said animal, which contains stem cells and includes, tissues and body fluids. "Body fluids" include blood and spinal fluid. "Tissue samples" include tissue obtained by surgical interventions (e.g. bone marrow or liver) or by other means e.g. placenta and umbilical cord. The animals from which cells are derived or to which the methods are applied are preferably as described' hereinbefore in connection with the methods of- reducing aberrant cell division.

As used herein reference to "improving the number of stem cells" refers to increasing the number of stem cells to be added (preferably of the particular type to be added, e.g. haematopoietic stem cells) relative to the number present in the individual at the time at which administration would occur. Thus in the case of a patient subject to chemotherapy or radiotherapy the observed improvement is in the number of stem cells in a patient post-chemotherapy or post-radiotherapy. An improvement may also consist of the addition of certain stem cells previously absent or present in very low numbers, e.g. neuronal stem cells.

Alternatively expressed, the present invention provides the use of an agent (preferably an antagonist as described hereinbefore) in the preparation of a medicament for the treatment or prevention of conditions or disorders typified by a need for stem cells, preferably in treating or preventing conditions or disorders in which stem cell numbers are lower than normal, e.g. due to chemotherapy or radiotherapy, or in conditions in which the provision of stem cells may allow the production of one or more particular differentiated cells that are absent or present in abnormally low numbers, or lower numbers than desired, at the site of interest.

Conditions or disorders in which stem cell numbers are lower than normal include autoimmune disorders, radiotherapy, chemotherapy and certain viral infections. Conditions in which the use of stem cells by transplantation may provide appropriate differentiated cells which are absent or present at lower than normal or lower than desired levels include Alzheimer's disease, Parkinson's disease and other age-related disorders or conditions (including cosmetic treatments), multiple sclerosis, spinal cord injury, diabetes, chronic heart disease, end-stage kidney disease, liver failure and in which stem cells are used to replace destroyed or dysfunctional cells. Prevention of such conditions or disorders may be achieved by maintaining stem cells in a protected state by the use of agents as described hereinbefore.

The present invention further provides the use of cells prepared by the methods described hereinbefore in the preparation of a medicament for the treatment of conditions or disorders typified by a need for stem cells, as described above.

It should be noted that due to the effects of the aforementioned agents on aberrant cell division, even samples of stem cells containing such aberrant cells may be used and a dual effect of reducing the aberrant division while expanding the stem cells may be achieved. Thus the aforementioned agents may be used *in vitro, ex vivo* or *in vivo* to protect normal stem/progenitor cells whilst eliminating cells undergoing aberrant cell growth. This is particularly applicable to haematopoietic cells, e.g. when treating leukaemia/lymphoma.

Thus in a further preferred aspect the present invention may be used for treating or preventing a condition or disorder in which aberrant cell division occurs. e.g. cancer, in a human or non-human animal, wherein said method comprises administering an agent, preferably an antagonist as described hereinbefore, wherein said agent is capable of both reducing said aberrant cell division and maintaining or expanding stem cells of said animal.

As described above, agents which reduce or prevent PBX-dependent transcription regulation, particularly HOX:PBX antagonists and particularly peptides as described hereinbefore have various clinical applications and thus a further aspect of the invention provides pharmaceutical compositions containing such agents. The use of these agents as a medicament forms a further aspect of the invention.

Thus, in a further aspect the present invention provides a pharmaceutical composition comprising an agent which reduces or prevents PBX-dependent transcription regulation as described hereinbefore preferably an antagonist, especially preferably an antagonist of the interaction between HOX and PBX, e.g. a peptide as described herein, or a polynucleotide or vector capable of expressing such a peptide, and a pharmaceutically acceptable excipient, diluent or carrier.

Pharmaceutical compositions as described herein for use as a medicament, preferably for use in treating or preventing disorders or conditions typified by aberrant cell division, or disorders or conditions typified by a need for stem cells, such as the conditions described herein, and methods of treatment or prophylaxis using such compositions and use of said agents for the preparation of a medicament for treating or preventing such disorders or conditions, form further aspects of the invention.

"Pharmaceutically acceptable" as referred to herein refers to ingredients that are compatible with other ingredients of the compositions as well as physiologically acceptable to the recipient.

Pharmaceutical compositions according to the invention may be formulated in conventional manner using readily available ingredients. Thus, the active ingredient (e.g. the peptide) may be incorporated, optionally together with other active substances, with one or more conventional carriers, diluents and/or excipients, to produce conventional galenic preparations such as tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, sterile packaged powders, and the like.

Compositions may additionally comprise molecules which assist or augment the action of the agents, preferably the peptides, described hereinbefore, e.g. cytotoxic agents such as antimetabolites, alkylating agents, cytotoxic antibiotics, topoisomerase I and/or II inhibitors, vinca alkaloids and monoclonal antibodies.

If required, the compositions may also contain targeting moieties attached to the active ingredient, e.g. a ligand which binds specifically and selectively to an endogenous receptor to allow targeting to a particular cell type or location, such as targeting to lymphocytes, monocytes, macrophages, endothelial cells, epithelial cells, blood cells, erythrocytes, platelets, eosinophils, neutrophils, natural killer cells, dendritic cells, brain cells, heart cells, lung cells, islet cells, kidney cells, cancer cells, hormonal gland cells, skin, bone, joints, bone marrow, gastric mucosa, lymph nodes, peyers patches, the omentum and other appropriate tissues.

Examples of suitable carriers, excipients, and diluents are lactose, dextrose, sucrose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/glycol, water/polyethylene glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The compositions of the invention may be formulated so as to provide quick, sustained or delayed release of the active ingredient after administration to the patient by employing procedures well known in the art.

The pharmaceutical carrier or diluent may be, for example, an isotonic _ solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

Compositions may be in an appropriate dosage form, for example as an emulsion or in liposomes, niosomes, microspheres, nanoparticles or the like.

Administration of agents or compositions of the invention may take place by any of the conventional routes, e.g. orally, rectally or parenterally, such as by intramuscular, subcutaneous, intraperitoneal or intravenous injection, infusion, inhalation or topical administration, both to internal or external body surfaces etc. depending on the condition or disorder to be treated or prevented, optionally at intervals, e.g. 3 or more applications at 3∼5 day intervals. Conveniently intravenous injection is used.

The active ingredient in composition of the invention may comprise from about. 0.01 % to about 99% by weight of the formulation, preferably from about 0.1 to about 50%, for example 10%. The compositions are preferably formulated in a unit dosage form, e.g. with each dosage containing from about. 0.01 mg to about 1g of the active ingredient, e.g. 0.05mg to 0.5g, for a human, e.g. 1-100mg.

The precise dosage of the active compound to be administered and the length of the course of treatment will, of course, depend on a number of factors including for example, the age and weight of the patient, the agent to be used, the purpose of the treatment, the specific condition requiring treatment or prevention and its severity, and the route of administration.

Generally however, an effective dose may lie in the range of from about 1µg/kg to about 10mg/kg, e.g. from about 1mg to 0.2g of the agent per day, depending on the animal to be treated and the dosage form, taken as a single dose. Thus for example, an appropriate daily dose for an adult may be from 0.5mg to 0.5g per day, e.g. 1 to 100mg of the polypeptide per day. In smaller animals the concentration range may be different and can be adjusted accordingly.

For *in vitro* or *ex vivo* use a concentration range of 1µg/ml to 10mg/ml for the agent, e.g. the peptide as described hereinbefore, is suitable.

Peptides of the invention may be used to assist or augment the action of agents used for conventional treatments, e.g. cytotoxic agents, to reduce their side effects, e.g. by protection of stem cells during treatment.

In one embodiment, a peptide of the invention is administered alongside one or more other therapeutically active agents. For example, a peptide of the invention may be used as a combinatorial chemotherapeutic agent. As shown in the Examples, peptides of the invention can induce some cancer cells, e.g. AML cells, to enter the cell cycle. Cells which have been stimulated in this way may therefore become more susceptible to conventional anti-cancer drugs. The peptides of the invention may therefore be used in combination with other anti-cancer agents, such as cytotoxic drugs, to target cancers such as leukaemia, more preferably acute myeloid leukaemia (AML) as explained above.

Peptides of the invention may also be used in combination with other anticancer therapies in order to protect the endogenous stem cell population. The inventors have discovered that the peptides of the invention are able to maintain normal stem/progenitor cells in a G0/G1 quiescent state. This cytoprotective ability may thus protect such stem cells from the effects of any anti-cancer treatment. This may be of particular use where the peptides of the invention are used in combination with cytotoxic agents which target dividing cells. By maintaining the normal stem cells of the patient in a quiescent state during such treatment, the side effects of the anti-cancer treatment on the endogenous stem cell population can be minimised. This reduction in the potential side effects may also allow a higher dose or level of the conventional treatment to be used on the patient than would otherwise be possible or safe.

Where a peptide of the invention is to be used in combination with or alongside one or more other therapeutic agents, for example an anti-cancer agent such as a cytotoxic drug, the agents may be formulated for simultaneous, sequential or separate administration. The agents may be formulated together in a single pharmaceutical composition. The agents may be formulated separately and may then be administered together, at the same time or sequentially, or at separate times during a course of treatment. Thus the peptides of the invention may be administered to a patient who has been administered, is being administered or is to be administered another therapeutic agent for example a cytotoxic or chemotherapeutic agent. The peptide may administered simultaneously with, sequentially with or separately to the other agent.

The following Examples illustrate the invention:

### Example 1: Effect of peptides on cell viability in vitro

In this example peptides generated to mimic the conserved region on HOX proteins, specifically the hexapeptide region of HOXB-4, were used in *in vitro* assays to determine their effects on cell death and cell proliferation in a variety of cell lines.

### Methods

### 1. Peptide design

To design a reagent that can prevent the interaction between PBX and HOX proteins, the highly conserved HOX hexapeptide sequence WYPWMKKHH (SEQ ID NO: 5) which is known to mediate this process (Morgan *et al.,* 2000) was linked to a polyarginine peptide previously shown to mediate efficient movement of proteins across cell membranes. This peptide is referred to herein as HXArg9 (SEQ ID NO: 6).

Further peptides were also constructed:

Peptide HXP4 (SEQ ID NO: 10) contains an identical HOX/PBX interfering peptide as HXArg9, but has a penetratin cell penetration sequence in place of polyarginine.

CX1Arg9 (SEQ ID NO: 7) was generated as a control peptide, based on the HXArg9 sequence but with a number of amino acid substitutions in the HOX/PBX interfering peptide sequence. CXP4 (SEQ ID NO: 11) is the equivalent control for the HXP4 sequence, containing the same HOX/PBX interfering peptide sequence attached to the penetratin cell penetration sequence.

CX2Arg9 (SEQ ID NO: 8) is a more extensively changed version of the control peptide CX1Arg9.

Arg9 (SEQ ID NO: 9) is simply a peptide consisting of 9 arginine residues on their own. This is intended as a control on possible non-specific toxicity.

The sequences of these peptides are as follows. All peptides were prepared by routine chemical synthesis.

Sequences having a polyarginine penetration sequence. Amino acid changes from the HXArg9 sequence are shown in bold type.

| | | |
|---|---|---|
| HXArg9 (SEQ ID NO: 6): | WYPWMKKHHRRRRRRRRR | Mass: 2700 . 06 Da |
| CX1Arg9 (SEQ ID NO: 7): | WCPW**LDR**H**G**RRRRRRRRR | Mass: 2555.87 Da |
| CX2Arg9 (SEQ ID NO: 8): | **MCAAGDDAL**RRRRRRRRR | Mass: 2251.75 Da |
| Arg9 (SEQ ID NO: 9): | RRRRRRRRR | Mass: 1405.71 Da |

### D-substituted variants (amino acids present in D-form underlined)

| | |
|---|---|
| HXArg9(2D) (SEQ ID NO: 12): | WYPWMKKHHRRRRRRRRR |
| CX1Arg9(2D) (SEQ ID NO: 13): | WCPWLDRHGRRRRRRRRR |
| CX2Arg9(2D) (SEQ ID NO: 14): | MCAAGDDALRRRRRRRRR |

Sequences having a penetratin based penetration sequence. Amino acid changes from the HXP4 sequence are shown in bold type.

| | |
|---|---|
| HXP4 (SEQ ID NO: 10): | WYPWMKKHHRQIKIWFQNRRMKWKK |
| CXP4 (SEQ ID NO: 11): | WCPW**LDR**HGRQIKIWFQNRRMKWKK |

### D-substituted variants (amino acids present in D-form underlined)

| | |
|---|---|
| HXP4(2D) (SEQ ID NO: 15): | WYPWMKKHHRQIKIWFQNRRMKWKK |
| HXP4(3D)1 (SEQ ID NO: 16): | WYPWMKKHHRQIKIWFQNRRMKWKK |
| HXP4(3D)2 (SEQ ID NO: 17): | WYPWMKKHHRQIKIWFQNRRMKWKK |
| HXP4(3D)3 (SEQ ID NO: 18): | WYPWMKKHHRQIKIWFQNRRMKWKK |
| HXP4(3D)4 (SEQ ID NO: 19): | WYPWMKKHHRQIKIWFQNRRMKWKK |

### 2. Cell lines culture

B16 mouse melanoma, LNCAP (human prostate cancer) and K562 (human acute myeloid leukaemia) cell lines were obtained from the ATCC (catalogue nos. CRL6475 Mus musculus, CRL1740 Homo sapiens and CCL243 Homo sapiens respectively).

Cell lines were seeded at 4x10⁴ cells/ml in liquid culture system made of RPMI- 1640 medium (Life Technologies, UK) supplemented with Foetal Calf Serum (10% Sigma Aldrich) and penicillin/streptomycin (1%, Sigma) and at least in duplicate. When required the test peptide was added to the medium. Cell lines were cultured for up to 7 days at 37°C, 5% CO₂ in humidified atmosphere. Cultured cells were then assessed for cell viability / enumeration using trypan blue exclusion (Sigma Aldrich).

For nuclear staining, cells were plated at a density of 9 x 10⁴ cells/ml in 25cm² culture flasks. After treatment for 48 hours, the cells were detached using Trypsin/EDTA slution (Sigma) and 200 µl aliquots were used to make cytospins at 800rpm for 5 minutes using a cytospin 4 (ThermoShandon)

### 3. cDNA synthesis

RNA was reverse transcribed as described previously. Briefly, RNA was first denatured by heating to 65°C for five minutes. 1-5µg of RNA was incubated in a volume of 50µl at 37°C for one hour with final concentrations of 10mM DTT, 1mM dNTPmix, as well as 100µg/µl polyt primers, 200 units of reverse transcriptase (Invitrogen, USA) and 40 units of RNASEout (Invitrogen, USA). The cDNA synthesis reaction was terminated by placing tubes at 65°C for five minutes.

### Results

### 1. L- and D- isomer peptides

D-isomer substitute of HXArg9 was generated having the N-terminal and C-terminal most bonds changed from the natural L-conformation to the D-conformation (hence this is a stereo-isomer, and has exactly the same chemical formula). We refer to this as HXArg9(2D). This peptide was used in a number of the cell line studies above, as indicated in the Figure legends.

A similar stereoisomer form of CX2Arg9 was also generated, in which the N-terminal and C-terminal most bonds are changed from the natural L-conformation to the D-conformations (CX2Arg9(2D)).

The stability of HXArg9(2D) and CX2Arg9(2D) was assessed by incubation each with human plasma at 37°C and the amount of peptide remaining after different amounts of time was assessed by SDS-PAGE. Each peptide was added to freshly prepared human plasma to a final concentration of 1mM in a total volume of 40µl. Samples were incubated at 37°C and reactions were stopped by adding 60µl of water and 0.9ml of protein gel loading buffer (BioRad, USA) containing 1% 2-mercaptoethanol, and heating to 95°C for four minutes. 2µl of each sample was run on a 22% acrylamide gel. This indicated that the half life of both HXArg9(2D) and CX2Arg9(2D) was approximately 12 hours (Figure 1). This compared with a half life of approximately 40 minutes for the L isomer form HXArg9.

As shown in Figure 8, the (2D) isomer form of the peptide (HXArg9(2D)) has equivalent activity to the L isomer form (HXArg9). Further studies have shown that HXArg9(2D) has an equivalent activity *in vitro* to HXP4 (B16 melanoma cells).

Experiments were also carried out with D-isomer variants of HXP4 (see Figures 2 and 3). The isomers having 3 D amino acids showed a similar level of activity and a similar half life to the isomer having 2 D amino acids (HXP4(2D) as above). The HXP4(3D)1 isomer showed slightly higher activity than the other 3D isomers. As shown in Figure 2B and 3, HXP4, HXP4(3D)2, HXP4(3D)3 and HXP4(3D)4 all significantly reduced cell growth of B16 cells over a 4 day period.

### 2. Specificity of HXArg9

In order to test the specificity of HXArg9, murine B16 melanoma cells were treated with 60µM HXArg9(2D) or CXArg9(2D) for one hour and protein was extracted from the cells. HOXD9, a known PBX binding partner that is expressed in melanoma (Svingen, T. & Tonissen, K.F. Cancer Biol. Ther. 2, 518-523 (2003)), was then immunoprecipitated using an anti-PBX antibody. This technique permits the recovery of HOXD9 in CXArg9(2D) - treated cells but not in HXArg9(2D) treated cells, indicating that HXArg9(2D) does indeed block the interaction between PBX and HOX (Figure 4A). As a further control on specificity, we also immunoprecipated the glucocorticoid receptor (GR). This binds to PBX through an interaction that does not depend on the presence of the hexapeptide motif in the GR protein (Subramaniam, N.et al. Biochem. J. 370, 1087-1095 (2003)), and thus this particular interaction should not be blocked by HXArg9(2D), and, indeed, it is not (Figure 4A).

As an additional test of the peptides' selectivity, binding to a double stranded DNA probe containing a HOX / PBX consensus binding site was studied. If the hexapeptide does prevent the interaction of HOX and PBX, it should also disrupt the binding of these proteins to HOX/PBX DNA consensus sites. In order to test this, a band-shift type assay was utilised.

B16 cells were treated with 60µM HXArg9(2D) or CXArg9(2D) for one hour, and then a cell-free extract was prepared. A streptavidin labelled DNA probe was added that contains either a HOX/PBX consensus site (HP) or a modified sequence that does not match the consensus site (CP; Figure 4B). The forward sequences of these oligos were: HP (containing a HOX/PBX consensus binding site) - GGACA AACTG AAGGC AGAGC TGATT TATGG CACAC ACACA AGAAT GGACA AACCC GTGAG, CP (containing an altered HOX/PBX binding site as a control) - GGACA AACTG AAGGC AGAGC GCTCC GTTAA CACAC ACACA AGAAT GGACA AACCC GTGAG.

The addition of the HP probe to the CXArg9(2D) or control B16 lysates, followed by resolution on a non-denaturing acrylamide gel resulted in a significant reduction in mobility. This indicates that one or more proteins had bound to it. In order to characterise these proteins, a 'super-shift' assay was performed, whereby an-anti PBX antibody was added to the lysate. Complexes were super shifted using the anti-PBX1,2,3 antibody (c-20; Santa Cruz, USA) at a dilution of 1:2000. This resulted in a further shift in mobility equivalent to a single antibody molecule binding, and indicating that each probe-binding protein complex contains a single PBX protein.

Importantly, whilst proteins in the cellular extracts from untreated and CXArg9(2D) treated cells bound to the probe, those from the HXArg9(2D) extracts did not. This suggests that HXArg9(2D) does indeed prevent the formation of HOX/PBX dimers and their subsequent binding to DNA.

### 3. HArg9(2D) peptide blocks the proliferation of a range of different malignant cell lines.

The activity of the peptides described above was tested on a number of different malignant cell lines using a similar assay to that described previously. The results are shown in Figures 6 to 8.

Apoptotic morphology was determined using scanning electron microscopy and fluorescence microscopy. Changes in nuclear morphology were assessed by fixing B-16 cytospins with ice-cold methanol at room temperature for five minutes. The fixed cells were stained with Hoechst 33258 (2µg/ml) (Sigma) and mounted using VECTASHIELD® mounting medium (Vector) and analyzed by fluorescence microscopy (Nikon Eclipse TE 2000-S).

Cells treated with 60µM HXArg9(2D) for 2 hours exhibited a notable change in morphology including a reduction in dendritic processes (Figure 5B). HXArg9(2D) treated cells also exhibited both a significant degradation of the genome, and characteristic changes in their ultrastructure such as nuclear breakdown and vacuolisiation (Figure 5C) typical of apoptosis. Staining with Hoechst dye also revealed a large number of HXArg9(2D)-treated cells displaying nuclear apoptotic morphology (chromatin condensation).

An additional sign of apoptosis is the appearance of phosphatidylserine in the cell membrane (Koopman, G. et al. Blood_84, 1415-1420 (1994)). This can be detected by fluorescencently labeled Annexin V protein and cell sorting using FACS.

Using this technique with B16F10 cells treated for two hours with 60µM HXArg9(2D) revealed that a significant proportion of cells were in either early or late phases of apoptosis. As a further demonstration that cell death was indeed through apoptosis, HXArg9(2D) treated B16 cells were co-treated with the Z-VAD peptide. This is a specific inhibitor of the caspase proteases, which are key components of the apotopic pathway (Zhu, H., et al.. FEBS Lett.374, 303-308 (1995)). The addition of Z-VAD caused a significant reduction in cell death when in combination with HXArg9(2D), indicating that the cells were indeed undergoing apoptosis. CX2Arg9(2D) had no effect on proliferation and did not cause apoptosis.

In addition to melanoma, a number of other malignancies are known to express HOX genes at significantly higher levels than the tissues from which they are derived (reviewed by Morgan, R. Trends Genet 22, 67-69 (2006)). These include prostate (Miller, G.J . et al. Cancer Res._63, 5879-5888 (2003)), renal (Cillo, C. et al. Int. J. Cancer. 51, 892-897 (1992)), bladder (Cantile, M ., et al. Oncogene 22, 6462-6468 (2003)), lung (Abe, M. et al. Oncol. Rep. 15, 797-802 (2006)) and ovarian (Cheng, W ., et al. Nat. Med. 11, 531-537 (2005)) cancer. In order to determine whether HXArg9 could also block the growth of cells derived from these cancers, we obtained the IC50 using an MTT assay for representative cell lines of each (Table 1). This revealed that all of these lines are sensitive to HXArg9(2D), with the non small cell lung cancer derived line A549 showing the highest level of sensitivity. A 60µM dose or a 6µM dose of HXArg9(2D) was sufficient to ablate the majority of cells derived from melanoma, prostate cancer and acute myeloid leukaemia.

In these experiments, HXP4 showed no effect. However, this peptide has previously been shown to be effective in similar assays. Further experiments showed that HXArg9 is as effective as HXP4 in preventing cellular proliferation, but works in a considerably shorter length of time (the assays reported in Figures 6 to 8 required 3 hour exposures; HXP4 typically requires 4 days to achieve the same response). The improved performance of HXArg9 is believed to be due to increased uptake efficiency from the presence of the polyarginine, rather than penetratin, cell penetration sequence.

This improved efficiency of polyarginine compared with penetratin is also seen in the results for the control peptides. The control peptide CX1Arg9 did show some activity. This is probably due to the relatively close sequence homology between the HOX/PBX interfering peptide sequence of HXArg9 (the "HX" sequence) and the equivalent sequence in this control peptide (the "CX" sequence). This contrasts with the related peptide CXP4 which comprises the "CX" sequence linked to penetratin and has previously been shown to be ineffective in a similar assay, even in the time scale in which HXP4 shows an effect.

A more heavily substituted control peptide (CX2Arg9) has no effect on cell proliferation.

**Table 1**

| **Cell Line** | **Source** | **IC50 (µM)** | |
|---|---|---|---|
| | | **HX** | **CX** |
| A549 | Human non-small cell lung carcinoma | 19 | 240 |
| LNCaP | Human prostate cancer | 21 | 220 |
| B16F10 | Mouse melanoma | 20 | 200 |
| SK-OV3 | Human ovarian cancer | 22 | 270 |
| A498 | Human renal cancer | 24 | 280 |
| J82 | Human bladder cancer | 22 | 290 |

### 4. Effect of peptide concentration

It is clear from Figures 6 to 8 that use of the HXArg9 at 6 and 60µM has an effect on the number of living cells in such cell lines. The dose responsiveness of such cells to HXPArg9 was therefore assessed.

As shown in Figure 9, a dose of 37µM or 74µM HXArg9 led to ablation of substantially all B16 murine melanoma cells. A dose as low as 4.625µM HXArg9 led to significant ablation of B16 murine melanoma cells.

### Example 2: Cross-reactivity of peptides with PBX proteins

### Methods

KG1a cells are grown under conditions as described in Example 1. KG1a cells are cultured for 24 hours without treatment, or in the presence of 1µM test peptide. The cells are harvested and lysed. One aliquot of the cells is subject to cross-linking. Frozen cells were lysed using standard techniques and 100µl of lysate is incubated for 30 minutes at room temperature in 4mM 1-ethyl-3-[3-(dimethyl-amino)propyl] carbodimide (EDC), 4mM sulpho-NHS, 20 mM HEPES (pH 7.5), 5 mM MgCl₂, and 0.03% (w/v) f3-DM to cross-link non-covalently associated proteins. The reaction is stopped by the addition of ammonium acetate to a final concentration of 50 mM. The other aliquot is frozen without cross-linking.

Proteins are extracted from the cells of both aliquots by standard techniques and separated by gel electrophoresis. The gels are probed by Western blot with an antibody raised against PBX1, 2 and 3 (sc-888, Santa Cruz Inc. USA) or an anti-beta actin antibody (sc-1615, Santa Cruz Inc. USA).

The PBX proteins from untreated and control peptide treated cells should associate with-other proteins (i.e. HOX proteins). Where a peptide capable of blocking the HOX-PBX interaction is used, the PBX isoforms from the peptide treated cells are not associated with other proteins. The effect of the peptide on the various PBX proteins indicates whether the peptide has a partial effectiveness or a global effect on all PBX proteins and is thus suitable to prevent the interaction between all PBX and HOX proteins.

### Example 3: Transcriptional chances caused by HXArg9(2D)

In order to examine changes in gene transcription upon HXArg9(2D) treatment, cells were treated for two hours with 60µM HXArg9(2D) or CX2Arg9(2D). RNA was extracted for analysis by Microarray, using the Mouse Genome 430A 2.0 Array (Affymetrix, USA), which contains 14, 000 characterized mouse genes. Whilst the majority of genes failed to show any significant changes in transcription, 22 showed an increase in transcription with a probability value of <0.05 (Table 2). Most notably, these included oncogenes *Fos* and *Jun.* In order to confirm that these genes are upregulated in HXArg9(2D)-treated B16 cells, quantitative PCR (QPCR) was used to measure the relative number of transcripts in RNA extracted from cells (Figure 10A); this confirmed that *Fos, Jun, Dusp1* and *Atf1* are all significantly upregulated in response to HXArg9(2D).

Quantitative RT-PCR was performed using the Stratagene MX4000 Real Time PCR machine. The Stratagene MX4000 measures PCR product accumulations during the exponential phase of the reaction, prior to the amplification becoming vulnerable to limited reagents and cycling variability. Fluorescence increases in accordance with increasing levels of PCR product.

**Table 2**

| **Gene name** | **Entrez reference** | **Product** | **T-test P-value** |
|---|---|---|---|
| *Drak2* | AY092028 | Serine/threonine kinase | 0.05 |
| *Klf4* | BG069413 | Transcription factor | 0.0439 |
| *Max* | NM_008558 | Transcription factor | 0.0407 |
| *Atf3* | NM_007498 | Transcription factor | 0.0275 |
| *Smad7* | NM_008543 | Signalling pathway | 0.0267 |
| *Jun* | NM_010591 | Transcription factor | 0.0141 |
| *Ski1* | NM_001039090 | Component of histone deacetylase complex | 0.00969 |
| *Dusp1* | NM_013642 | Phosphatase | 0.00955 |
| *Fos* | BC029814 | Transcription factor | 0.00274 |

As the relative amount of *cFos* RNA is elevated in HXArg9(2D)-treated cells we also examined the amounts of cFOS protein by western blotting with a cFOS specific antibody (Figure 11). In contrast to *cfos* RNA, the amount of cFOS protein in these cells was significantly reduced.

In this study we have shown that antagonising the interaction between HOX and PBX proteins triggers apoptosis in a number of different malignant cells, including murine B16 melanoma cells. The former occurs both *in vitro* and *in vivo,* and involves a common set of target genes that become dysregulated in these cells, including the oncogene *cfos. cfos* RNA increases significantly upon HXArg9(2D) treatment, and yet the level of cFOS protein is greatly reduced. This seemingly paradoxical finding probably reflects the close coupling that is known to occur between cFOS translation and the *cFos* RNA degradation (Veyrune et al., 1996). Thus an inhibition of cFOS translation would effectively stabilize *cFos* RNA.

*Fos* and its binding partner *Jun* are both members of the bZIP superfamily of transcription factors, which are characterized by a basic DNA-binding domain combined with a leucine zipper region (Hess, J et al. J. Cell Sci. 117, 5965-5973 (2004)). JUN can homodimerise to form the AP-1 transcription factor. FOS cannot homodimerise, but it too can form part of the AP-1 transcirption factor by binding JUN. AP-1 activates the expression of a number of genes involved in cell cycle regulation, including cyclin D1 (Albanese, C, et al. J. Biol. Chem. 270, 23589-23597 (1995)). Furthermore the inhibition of AP-1 triggers apoptosis in HL60 cells (Park, S.,et al.. J. Cell Biochem. 91, 973-986 (2004)) through a c-myc dependant mechanism that leads to the upregulation of pro-apoptopic genes.

In addition to cFos, a number of other target genes are also upregulated by HXArg9(2D), of which Dusp1 and Atf3 show by far the greatest increase. The former encodes a dual specificity phosphatase, a protein that can remove phosphate residues from serine, threonine and tyrosine residues of a wide range of substrates and can block signaling through the mitogen activated kinase pathway (1VIAPK), thereby blocking cellular proliferation (reviewed by Ducruet, A.P.,et al. Annu. Rev. Pharmacol. Toxicol. 45, 725-750 (2005)). Atf3 is a member of the ATF/CREB family of transcription factors that are known for their role in stress response, including DNA damage (Wek, R.C .et al.. Biochem. Soc. Trans. 34, 7-11 (2006)). It has recently been demonstrated that Atf3 can stabalise p53 by preventing its ubiquination (Yan, C. et a/.. EMBO J. 24, 2425-2435 (2005)), and promote its tumour suppressor functions, including the induction of apoptosis (Yan, C. & Boyd, D.D. Cell Cycle 5, 926-929 (2006)). Atf3 also promotes cell cycle arrest and apoptosis in UV treated fibroblast cells, and blocks Ras-mediated transformation (Lu, D. et al J. Biol. Chem. 281, 10473-10481 (2006)).

Many of the other target genes identified in the screen also have anti-tumour properties. Klf4 (Kruppel-like factor 4) is a transcription factor that is known to be down-regulated in many colorectal cancers, inhibits the activity of the oncogenic transcription factor β-catenin, and prevents xenograft tumour growth in athymic nude mice (Zhang, W . et al. Mol. Cell Biol. 26, 2055-2064 (2006)). Mad is an inhibitor of another oncogenic transcription factor, myc, and is also has a role in blocking proliferation (reviewed by Rottmann, S . & Luscher, B . Curr. Top. Microbiol. Immunol. 302, 63-122 (2006)). Finally, Drak2 is a serine/threonine kinase with a high degree of sequence and functional homology to the death associated kinases involved in apoptosis, and can induce apoptosis when over expressed in NIH 3T3 cells (Sanjo, H.,et al. J. Biol. Chem. 273, 29066-29071 (1998)).

The pre-dominance of tumour suppressor and pro-apoptotic genes amongst HXArg9(2D) targets, together with the ability of HXArg9(2D) to induce apoptosis in a number of different malignancies, suggest that the function of HOX gene expression in these cells may itself be to inhibit the intrinsic tumour suppressing pathways. The relative importance of these targets may vary from one cell type to another depending on the genetic background, but a generalized inhibition of many tumour suppressing pathways could account for the wide range of tumours that over express HOX genes. Together with the data presented here showing that HXArg9(2D) triggers apoptosis in a range of different tumour types, it can be seen that the HOX genes present a potentially important target in cancer therapy.

### Example 4: Effects af HXArg9 in vivo

Mice were maintained at the Biological Resource Facility at St.George's. Their care was in accordance to Home Once regulations. B16 cells in exponential growth phase were injected subcutaneously at a dose of 1x10⁶ cells into C57black/6 mice. Tumour growth was monitored daily. Once tumours were established, mice received 15mg/kg HXArg9(2D) intraperitoneal. Mice were euthanised and tumours excised and fixed for histopathological analysis.

Control groups received control peptide and PBS accordingly. For the A549 tumour model, NOD / SCID immune deficient mice were used to establish flank models. This study was conducted by Perry Scientific Inc., USA.

The B16 F10 murine tumour is a well established in vivo model of melanoma, and is also one of the most aggressive murine tumours (Fidler, I.J. & Nicolson, G.L. Isr. J. Med Sci. 14, 38-50 (1978), Shrayer, D. et al. Cancer Immunol. Immunother. 40, 277-282 (1995)). Subcutaneous inoculation was used to introduce 1 x 10⁵ cells, and mice were treated with intraperitoneal injections of HXArg9 or CX2Arg9 at 15mg/kg once tumours were palpable. At the end point of these experiments HXArg9 treated tumors showed a significant degree of shrinkage (Figure 12A), and histological analysis of excised tumours revealed inner cell death only in HXArg9 treated mice (Figure 12B).

In order to assess whether the hexapeptide could block HOX / PBX binding *in vivo,* tumours were treated with HXArg9 for two hours and then used to produce nuclear lysates. Band shift assays (as detailed above) revealed that protein extracted from these tumours could not bind a DNA probe containing a HOX/PBX consensus binding site, whereas an equivalent extract from CX2Arg9 treated tumours could (Figure 4C), indicating that HXArg9 specifically disrupts the formation of HOX/PBX/DNA complexes *in vivo.* QPCR analysis of RNA extracted from these tumors revealed that *Fos, Jun, Dusp1* and *Atf3* were upregulated, mirroring those results obtained *in vitro* (Figure 10B).

We also examined whether HXArg9 could block the growth of a non-melanoma tumour *in vivo.* In order to do this, we established flank tumours using the non-small cell lung cancer derived cell line A549 (Lieber, M., et al. Int. J. Cancer 17, 62-70 (1976)) in nude mice. Treatment with HXArg9 twice weekly at doses of 100mg/kg (first three treatments) followed by 10mg/kg caused a significant reduction in tumour growth (Figure 13).

## Claims

1. A peptide comprising the amino acid sequence (I):
Y₁ X₁ X₂ X₃ W X₄ X₅ X₆X₇ X₈ Y₂ (I)
wherein
X₁ is W;
X₂ is Y;
X₃ is P;
X₄ is M, I, V or L;
X₅ is K or R;
X₆ is K or R;
X₇ is H or T;
X₈ is one or more amino acids or absent; and
Y₁ and Y₂ are each either absent or (Arg)₉, such that at least one of Y₁ and Y₂ is present;
for use in treating or preventing a disorder in which aberrant cell division occurs.

2. A peptide according to claim 1 wherein at least the N-terminal and C-terminal amino acids of said peptide is in the D-conformation.

3. A peptide according to any one of the preceding claims wherein said sequence X₁ to X₈ is WYPWMKKHH or WYPWMKKHHR.

4. A peptide according to any one of claims 1 to 3, which has the sequence WYPWMKKHHRRRRRRRRR.

5. Use of a peptide as defined in any one of the preceding claims in the manufacture of a medicament for treating or preventing a disorder in which aberrant cell division occurs.

6. A peptide according to any one of claims 1 to 4 or use according to claim 5, wherein said disorder is a cancer.

7. A peptide according to any one of claims 1 to 4 or use according to claim 5, wherein said cells express one or more Hox genes.

8. A peptide according to any one of claims 1 to 4 or use according to claim 5, wherein PBX does not act as an oncogene in said cells.

9. Products containing a peptide as defined in any one of claims 1 to 4 and a cytotoxic or chemotherapeutic agent as a combined preparation for simultaneous, sequential or separate use in the treatment or prevention of a disorder in which aberrant cell division occurs.

10. Use of a peptide as defined in any one of claims 1 to 4 in the manufacture of a medicament for treating or preventing a disorder in which aberrant cell division occurs, wherein the patient is also to be administered a cytotoxic or chemotherapeutic agent.

11. Use of a cytotoxic or chemotherapeutic agent in the manufacture of a medicament for treating or preventing a disorder in which aberrant cell division occurs, wherein the patient is also to be administered a peptide as defined in any one of claim 1 to 4.

12. A peptide as defined in any one of claims 1 to 4 for use in reducing the side effects of a cytotoxic or chemotherapeutic agent.

13. A method of maintaining or expanding stem cells *ex vivo* comprising contacting said stem cells with a peptide as defined in any one of claims 1 to 4.

14. A pharmaceutical composition comprising a peptide as defined in any one of claims 1 to 4 and a pharmaceutically acceptable carrier.

15. A pharmaceutical composition according to claim 14 further comprising a cytotoxic or chemotherapeutic agent.

## Patentansprüche

1. Peptid, umfassend die Aminosäuresequenz (I):
Y₁X₁X₂X₃WX₄X₅X₆X₇X₈Y₂ (I)
wobei gilt:
X₁ ist W;
X₂ ist Y;
X₃ ist P;
X₄ ist M, I, V oder L;
X₅ ist K oder R;
X₆ ist K oder R;
X₇ ist H oder T;
X₈ ist eine oder mehrere Aminosäuren oder ist nicht vorhanden; und
Y₁ und Y₂ sind jeweils entweder nicht vorhanden oder sind (Arg)₉, so dass mindestens einer der Reste Y₁ und Y₂ vorhanden ist;
zur Verwendung bei der Behandlung oder Vorbeugung einer Erkrankung, bei welcher eine anomale Zellteilung stattfindet.

2. Peptid nach Anspruch 1, wobei mindestens die N-terminalen und die C-terminalen Aminosäuren des Peptids in der D-Konformation vorliegen.

3. Peptid nach einem der vorangegangenen Ansprüche, wobei die Sequenz X₁ bis X₈ W Y P W M K K H H oder W Y P W M K K H H R ist.

4. Peptid nach einem der Ansprüche 1 bis 3, welches die Sequenz W Y P W M K K H H R R R R R R R R R hat.

5. Verwendung eines Peptids, wie in einem der vorangegangenen Ansprüche definiert, bei der Herstellung eines Medikaments zur Behandlung oder Vorbeugung einer Erkrankung, bei welcher eine anomale Zellteilung stattfindet.

6. Peptid nach einem der Ansprüche 1 bis 4 oder Verwendung nach Anspruch 5, wobei die Erkrankung ein Krebs ist.

7. Peptid nach einem der Ansprüche 1 bis 4 oder Verwendung nach Anspruch 5, wobei die Zellen ein oder mehrere Hox-Gene exprimieren.

8. Peptid nach einem der Ansprüche 1 bis 4 oder Verwendung nach Anspruch 5, wobei PBX in den Zellen nicht als ein Onkogen wirkt.

9. Produkte, die ein Peptid, wie in einem der Ansprüche 1 bis 4 definiert, und ein zytotoxisches oder chemotherapeutisches Mittel als kombiniertes Präparat zur gleichzeitigen, aufeinander folgenden oder getrennten Verwendung bei der Behandlung oder Vorbeugung einer Erkrankung, bei welcher eine anomale Zellteilung stattfindet, enthalten.

10. Verwendung eines Peptids, wie in einem der Ansprüche 1 bis 4 definiert, bei der Herstellung eines Medikaments zur Behandlung oder Vorbeugung einer Erkrankung, bei welcher eine anomale Zellteilung stattfindet, wobei dem Patienten auch ein zytotoxisches oder chemotherapeutisches Mittel verabreicht werden soll.

11. Verwendung eines zytotoxischen oder chemotherapeutischen Mittels bei der Herstellung eines Medikaments zur Behandlung oder Vorbeugung einer Erkrankung, bei welcher eine anomale Zellteilung stattfindet, wobei dem Patienten auch ein Peptid, wie in einem der Ansprüche 1 bis 4 definiert, verabreicht werden soll.

12. Peptid, wie in einem der Ansprüche 1 bis 4 definiert, zur Verwendung beim Verringern der Nebenwirkungen eines zytotoxischen oder chemotherapeutischen Mittels.

13. Verfahren zum Erhalten oder Vermehren von Stammzellen ex *vivo,* umfassend das In-Kontakt-Bringen der Stammzellen mit einem Peptid, wie in einem der Ansprüche 1 bis 4 definiert.

14. Arzneimittel, enthaltend ein Peptid, wie in einem der Ansprüche 1 bis 4 definiert, und einen pharmazeutisch verträglichen Träger.

15. Arzneimittel nach Anspruch 14, das ferner ein zytotoxisches oder chemotherapeutisches Mittel enthält.

## Revendications

1. Peptide comprenant la séquence d'aminoacides (I) :
Y₁ X₁ X₂ X₃ W X₄ X₅ X₆ X₇ X₈ Y₂ (I) dans laquelle
X₁ est W ;
X₂ est Y ;
X₃ est P ;
X₄ est M, I, V ou L ;
X₅ est K ou R ;
X₆ est K ou R ;
X₇ est H ou T ;
X₈ est un ou plusieurs aminoacides ou est absent ; et
Y₁ et Y₂ sont chacun soit absents soit (Arg)₉, de façon à ce qu'au moins l'un de Y₁ et Y₂ est présent ;
pour une utilisation dans le traitement ou la prévention d'un trouble dans lequel une division cellulaire aberrante apparaît.

2. Peptide selon la revendication 1, dans lequel au moins les aminoacides N-terminales et C-terminales dudit peptide est de configuration D.

3. Peptide selon l'une quelconque des revendications précédentes, dans lequel ladite séquence X₁ à X₈ est W Y P W M K K H H ou W Y P W M K K H H R.

4. Peptide selon l'une quelconque des revendications 1 à 3, qui a la séquence W Y P W M K K H H R R R R R R R R R.

5. Utilisation d'un peptide tel que défini dans l'une quelconque des revendications précédentes pour la fabrication d'un médicament pour le traitement ou la prévention d'un trouble dans lequel une division cellulaire aberrante apparaît.

6. Peptide selon l'une quelconque des revendications 1 à 4 ou utilisation selon la revendication 5, ledit trouble étant un cancer.

7. Peptide selon l'une quelconque des revendications 1 à 4 ou utilisation selon la revendication 5, lesdites cellules exprimant un ou plusieurs gènes Hox.

8. Peptide selon l'une quelconque des revendications 1 à 4 ou utilisation selon la revendication 5, dans lequel ou dans laquelle PBX n'agit pas comme un oncogène dans lesdites cellules.

9. Produits contenant un peptide tel que défini dans l'une quelconque des revendications 1 à 4 et un agent cytotoxique ou chimiothérapeutique sous la forme d'une préparation combinée pour une utilisation simultanée, successive ou séparée dans le traitement ou la prévention d'un trouble dans lequel une division cellulaire aberrante apparaît.

10. Utilisation d'un peptide tel que défini dans l'une quelconque des revendications 1 à 4 dans la fabrication d'un médicament pour le traitement ou la prévention d'un trouble dans lequel une division cellulaire aberrante apparaît, dans laquelle il est aussi administré un agent cytotoxique ou chimiothérapeutique au patient.

11. Utilisation d'un agent cytotoxique ou chimiothérapeutique dans la fabrication d'un médicament pour le traitement ou la prévention d'un trouble dans lequel une division cellulaire aberrante apparaît, dans laquelle il est aussi administré au patient un peptide tel que défini dans l'une quelconque des revendications 1 à 4.

12. Peptide tel que défini dans l'une quelconque des revendications 1 à 4 pour une utilisation pour réduire les effets secondaires d'un agent cytotoxique ou chimiothérapeutique.

13. Procédé de maintien ou d'expansion de cellules souches *ex vivo,* comprenant l'amenée desdites cellules souches au contact d'un peptide tel que défini dans l'une quelconque des revendications 1 à 4.

14. Composition pharmaceutique comprenant un peptide tel que défini dans l'une quelconque des revendications 1 à 4 et un véhicule pharmaceutiquement acceptable.

15. Composition pharmaceutique selon la revendication 14, comprenant en outre un agent cytotoxique ou chimiothérapeutique.
